# EUROPÄISCHE PATENTSCHRIFT

(11) **EP 1 019 417 B1**
(45) Veröffentlichungstag und Bekanntmachung des Hinweises auf die Patenterteilung: **16.10.2002**
(21) Anmeldenummer: 98945258.6
(22) Anmeldetag: 18.08.1998
(51) Int. Cl.: C07F 9/10, C07F 9/09, A61K 9/127, C12N 15/88

(54) **PHOSPHOLIPIDANALOGE VERBINDUNGEN**
PHOSPHOLIPID ANALOGUE COMPOUNDS
COMPOSES ANALOGUES DE PHOSPHOLIPIDES

(30) Priorität: 18.08.1997 DE 19735776
(43) Veröffentlichungstag der Anmeldung: 19.07.2000
(60) Teilanmeldung: 02011757.8
(73) Patentinhaber: Max-Planck-Gesellschaft zur Förderung der Wissenschaften e.V., 80539 München (DE)
(72) Erfinder: EIBL, Hans-Jörg, D-37120 Bovenden (DE)
(74) Vertreter: Huber, Bernhard, Dipl.-Chem.
(86) Internationale Anmeldenummer: EP9805252
(87) Internationale Veröffentlichungsnummer: WO99009037

(56) Entgegenhaltungen:
- EP-A- 0 002 202
- WO-A-97/30058
- DD-A- 240 020
- MARUYAMA K ET AL: "PHOSPHATIDYL POLYGLYCEROLS PROLONG LIPOSOME CIRCULATION IN VIVO" INTERNATIONAL JOURNAL OF PHARMACOGNOSY, Bd. 111, 1994, Seiten 103-107, XP000672277
- ALLEN T M: "STEALTH LIPOSOMES: FIVE YEARS ON" JOURNAL OF LIPOSOME RESEARCH, Bd. 2, Nr. 3, 1. Januar 1992, Seiten 289-305, XP000303894

## Beschreibung

Die Erfindung betrifft neue phospholipidanaloge Verbindungen, die als Liposomenbestandteile zum Transport von Arzneimitteln, als Lösungsvermittler für in Wasser schlecht lösliche Arzneimittel und auch als Wirkstoffe selbst gegen Erkrankungen wie Krebs und Leishmaniose eingesetzt werden können, diese neuen Verbindungen enthaltende Liposomen, Arzneimittelzusammensetzungen, welche diese Liposomen enthalten, und Verfahren zur Herstellung von Arzneimitteln.

Insbesondere betrifft die Erfindung Phosphatidylverbindungen, die einen definierten hydrophilen Rest enthalten, sowie Liposomen, die eine verkürzte oder verlängerte Lebensdauer im Serum aufweisen und gezielt von bestimmten Zellen, beispielsweise Tumorzellen, aufgenommen werden können.

Herkömmliche Liposomen weisen im Serum eine Verweilzeit von bis zu 5 Stunden auf. Insbesondere bei der Verwendung von Liposomen als Träger für pharmazeutische Wirkstoffe ist jedoch eine möglichst lange Verweilzeit von Liposomen im Blutkreislauf wünschenswert, insbesondere aber in Verbindung mit einer Aufnahme in ausgewählte Zielzellen.

Es wurden daraufhin die sogenannten "Stealth-Liposomen" entwickelt, die eine verlängerte Lebensdauer aufweisen. Diese "Stealth-Liposomen" sind auf der Basis von Phosphatidylverbindungen aufgebaut, die einen verlängerten Polyethylenglykolrest enthalten. Der Polyethylenglykolrest erwies sich für die angestrebte verlängerte Lebensdauer am wirksamsten bei Molekulargewichten zwischen 2000 und 3000. Ein wesentlicher Nachteil dieser "Stealth-Liposomen" bzw. der Phosphatidylverbindungen mit Polyethylenglykolrest liegt allerdings darin, dass es sich nicht um exakt definierte Verbindungen handelt, da die Polyethylenglykolreste unterschiedliche Kettenlängen aufweisen. Die sogenannten "Stealth-Liposomen" besitzen infolge des Phosphatrestes jedoch immer eine negative Oberflächenladung in der Liposomenmembran. Aufgabe der eigenen älteren Patentanmeldung 196 22 224.9 war deshalb die Bereitstellung von Verbindungen, die die Lebensdauer von Liposomen erhöhen und eine exakt angebbare Zusammensetzung aufweisen, wobei ebenfalls Phosphatester und damit negative Ladungen verwendet wurden.

Aus der eigenen älteren Anmeldung EP 0 002 202 sind Phospholipide bekannt, die eine positive Überschussladung aufweisen durch Überkompensation der negativen Ladung am Phosphat aufgrund der Einführung von zwei Stickstoffatomen. Durch diese positive Überschussladung wird jedoch die Serumstabilität damit hergestellter Liposomen herabgesetzt. Ein Hinweis, wie die negative Überschussladung ohne Einführung positiver Überschussladungen vermieden werden und für die Herstellung serumstabiler Liposomen besonders geeignete Phospholipide erhalten werden können, lässt sich daraus nicht entnehmen.

Aus der DD 240 020 A1 ist ein Verfahren zur Herstellung pharmakologisch wirksamer Phospholipide bekannt, welches zu racemischen Verbindungen führt. Optisch reine Verbindungen, wie sie für die Liposomenherstellung erwünscht sind, lassen sich nach diesem Verfahren nicht erhalten.

Aufgabe der vorliegenden Erfindung ist es dagegen, Verbindungen bereitzustellen, welche diese negative Oberflächenladung vermeiden und die Oligoglycerin- oder Zuckerreste über ein Stickstoffatom in die Struktur einbinden. Durch die positive Ladung am Stickstoffrest wird die negative Ladung am Phosphat ausgeglichen oder sogar überkompensiert, wenn 2 Stickstoffatome im Molekül verwendet werden. Diese Aufgabe wird erfindungsgemäß gelöst durch eine Verbindung einer allgemeinen Formel I 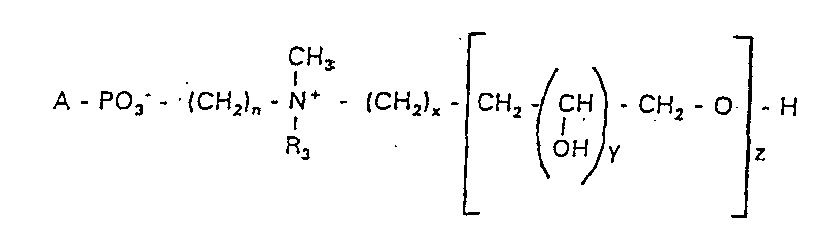 worin A 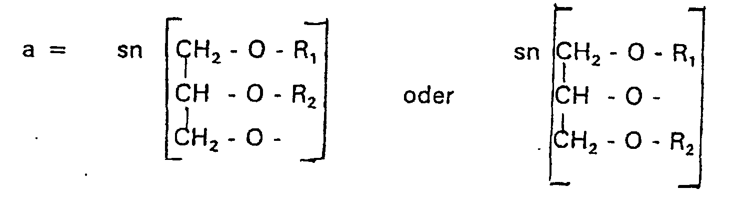 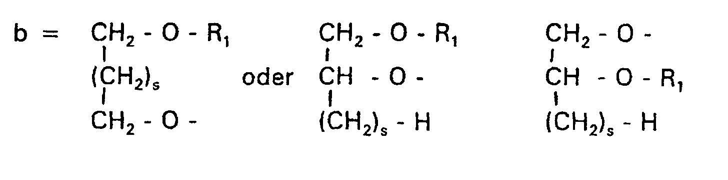 ist, wobei R₁ und R₂ unabhängig voneinander Wasserstoff, einen gesättigten oder ungesättigten Acyl- oder Alkylrest bedeuten, der gegebenenfalls verzweigt oder/und substituiert sein kann, wobei die Summe der Kohlenstoffatome in Acyl und Alkyl 16 bis 44 C-Atome beträgt,
- s: eine ganze Zahl von 0 bis 8 darstellt,
- c =: ein Rest eines primären oder sekundären Alkohols der Formel RO- ist, wobei R einen gesättigten oder ungesättigten Alkylrest vorwiegend mit cis-Doppelbindung von 12 bis 30 Kohlenstoffatomen bedeutet,
- n: eine ganze Zahl von 2 bis 8 darstellt,
- R₃ a =: 1,2-Dihydroxypropyl sein kann oder
- b =: Alkyl mit 1 bis 3 C-Atomen sein kann, wenn z > 0 ist oder
- c =: Alkyl mit 1 bis 3 C-Atomen sein kann, wenn n ≠ 2 und z = 0 ist,
- x =: eine ganze Zahl von 0 bis 8 darstellt,
- y =: 1 für z = 1 bis 5 ist oder
- =: 1 bis 4 ist für z = 1
- z =: eine ganze Zahl von 0 bis 5 darstellt.

Es sind Verbindungen bevorzugt, bei denen nicht gleichzeitig 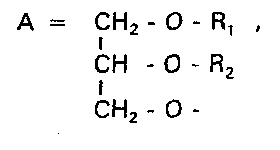 n = 2, x = 0, z ≠ 0 ist und R₃ einen Alkylrest darstellt.

Die in den hier beschriebenen Substanzen verwendeten Strukturelemente können beliebig variiert und maßgeschneidert der jeweiligen Verwendung angepasst werden.

Über den Strukturparameter A können vorwiegend die apolaren Anteile der Moleküle variiert werden, z.B. über die Kettenlänge der Fettsäuren und der Alkylreste. Eine Modifikation der polaren Anteile ist über die Phosphatgruppe, das Stickstoffatom und die damit verknüpften Oligoglycerine möglich.

Die durch die allgemeine Formel I erfassten Verbindungen besitzen hervorragende biologische Eigenschaften und finden Verwendung als
1) Liposomenbestandteile zur gezielten Anreicherung von Wirkstoffen in Zielzellen,
2) Lösungsvermittler für schwer intravenös applizierbare Substanzen, wie z.B. Taxol,
3) Wirkstoffe gegen Krebs und Protozoenerkrankungen.

Die Verbindungen, die in den verschiedenen Anwendungsbereichen besondere Bedeutung besitzen, werden nun im Detail beschrieben. Dabei gibt es Überschneidungen, da disubstituierte Glycerine mit dem Strukturmerkmal A sowohl membranstabilisierende Eigenschaften (R₁ + R₂ > 20 C-Atome) wie auch membrandestabilisierende Eigenschaften (R₁ + R₂ < 20 C-Atome) besitzen können. Insbesondere die Grenzbereiche zwischen Membran- und Mizellbildnern können hier von besonderem Interesse sein.

Allen Strukturen gemeinsam ist die einfache Herstellung der neuartigen Moleküle, die durch Umsetzung von primären oder sekundären Aminen mit Epoxiden erfolgen kann. So entsteht aus 1,2-Dipalmitoyl-sn-glycero-3-phospho-(N-methyl)-ethanolamin mit Benzylglycidol nach katalytischer Debenzylierung und Methylierung am Stickstoff ein Phospholipid mit lecithinartiger Struktur, das als Liposomenbestandteil verwendet wird.

Verbindungen mit nur einer langen Alkyl- oder Acylkette besitzen andere interessante Eigenschaften, wenn sie über primäre oder sekundäre Amine mit Epoxiden verknüpft werden, wie aus nachfolgender Beschreibung hervorgeht. Es sind ausgezeichnete Lösungsvermittler für schlecht intravenös applizierbare Wirkstoffe und sogar direkte Wirkstoffe gegen Krebs und Leishmaniose.

Der dieser Erfindung zugrundeliegende schrittweise Aufbau der hydrophilen Reste der Phosphatidylverbindungen der Formel I ermöglicht es, eine genau definierte Zusammensetzung der Verbindungen zu erhalten.

Es handelt sich also bei der erfindungsgemäßen Verbindung der Formel I nicht um ein Gemisch verschiedener Moleküle unbestimmter Zusammensetzung und Kettenlänge, sondern es kann gezielt eine gewünschte Struktur erhalten werden. Dies bedeutet, falls das gewünschte Produkt ein N,N-Dimethyl-N-(2-hydroxypropyl-3,1-O,O-dihydroxypropyl)-ammoniumderivat ist mit y = 1 und z = 2 in der Formel I, dass die Verbindung chemisch definiert ist und keine Anteile mit y = 1 und z = 1 oder y = 1 und z = 3 usw. enthält. Bevorzugt werden Hydroxypropylderivate einer ganz bestimmten Kettenlänge verwendet, die im wesentlichen frei von anderen Kettenlängen sind.

Erfindungsgemäß stellt die Verbindung der Formel I eine einheitliche Verbindung definierter Struktur dar. Bevorzugt ist die Verbindung hinsichtlich des Wertes von z größer als 99 % einheitlich. Es ist jedoch auch möglich, die Verbindung mit einer Einheitlichkeit von mehr als 99,9 % hinsichtlich des Wertes von z bereitzustellen.

Bevorzugt handelt es sich bei der Verbindung um Hydroxypropylderivate am Stickstoff mit 1 bis 5 Hydroxypropyleinheiten, bevorzugt mit 1 bis 3 Hydroxypropyleinheiten. Es handelt sich dabei bevorzugt um 1,3-verknüpfte lineare Oligoglycerinreste, die über einen 2-Hydroxypropylrest mit dem Stickstoffatom verknüpft sind.

Der Rest für A = c mit der Formel RO- leitet sich von einem primären oder sekundären Alkohol ab. Wenn RO- von einem sekundären Alkohol stammt, sind solche Reste mit dem Sauerstoff am C₂-, C₃- oder C₄-Atom bevorzugt.

Die Reste R¹ und R² stellen erfindungsgemäß bevorzugt unabhängig voneinander Wasserstoff, einen gesättigten oder ungesättigten Acyl- oder Alkylrest dar, der gegebenenfalls verzweigt oder/und substituiert sein kann, wobei die Summe der Kohlenstoffatome in Acyl und Alkyl zwischen 16 und 44 liegt.

Ein weiterer Gegenstand der Erfindung sind Liposomen, die Phospholipide oder/und Alkylphospholipide, gegebenenfalls Cholesterin und 1 bis 50 Mol-% einer Verbindung der allgemeinen Formel I beinhalten.

Die Phospholipide oder/und Alkylphospholipide können beispielsweise Diacylglycerophosphoverbindungen definierter Struktur sein. Allgemein können diese Bestandteile der Lipide als Verbindungen definierter Struktur eingesetzt werden.

Im Falle y > 1 stammt der Rest -CH₂(CHOH)_{y} CH₂-OH bevorzugt aus Zuckeralkoholen, die für y = 2, 3 Hydroxylgruppen, für y = 3, 4 Hydroxylgruppen und für y = 4, 5 Hydroxylgruppen aufweisen. Beispiele solcher Reste sind Mannitderivate für y = 4, Lyxitderivate für y = 3 und Threitderivate für y = 2.

Die erfindungsgemäßen Liposomen weisen eine deutlich veränderte Halbwertszeit in der Blutzirkulation auf. Die Liposomen sind nach außen neutral und zeigen erhöhte Verweilzeiten im Blut.

Ein weiterer Gegenstand der Erfindung ist eine pharmazeutische Zusammensetzung, die die oben beschriebenen Liposomen und in den Liposomen eingeschlossen einen oder mehrere pharmazeutische Wirkstoffe gegebenenfalls zusammen mit pharmazeutisch üblichen Verdünnungs-, Hilfs-, Träger- und Füllstoffen enthält.

Als Wirkstoffe können in der Regel alle Wirkstoffe verwendet werden, die sich mittels Liposomen überhaupt ins Plasma einbringen lassen. Bevorzugte Wirkstoffgruppen sind einerseits Cytostatika, insbesondere Anthracyclin-Antibiotika, wie etwa Doxorubicin, Epirubicin oder Daunomycin, wobei Doxorubicin besonders bevorzugt ist. Weitere bevorzugte Cytostatika sind Idarubicin, Hexadecylphosphocholin, 1-Octadecyl-2-methyl-rac-glycero-3-phosphocholin, 5-Fluoruracil, cis-Platinkomplexe wie Carboplatin und Novantron sowie Mitomycine.

Weitere bevorzugte Wirkstoffgruppen sind immunmodulierende Substanzen wie etwa Cytokine, wobei unter diesen wiederum die Interferone und insbesondere das α-Interferon besonders bevorzugt sind, antimykotisch wirksame Substanzen (z.B. Amphotericin B) und Wirkstoffe gegen Protozoenerkrankungen (Malaria, Trypanosomen- und Leishmanien-Infektionen). Ebenfalls bevorzugt ist Taxol als Wirkstoff.

Eine weitere bevorzugte Wirkstoffgruppe sind lytische Wirkstoffe, wie sie in der DE 41 32 345 A1 beschrieben sind. Bevorzugt sind Miltefosin, Edelfosin, Ilmofosin sowie SRI62-834. Insbesondere bevorzugt sind Alkylphosphocholine auch mit erweiterten Alkylketten, z.B. Erucylphosphocholin und Erucylphosphocholine mit erweitertem Phospho-Stickstoffabstand.

Die erfindungsgemäßen Liposomen werden nach an sich bekannten Methoden hergestellt mit hierfür gängigen Vorrichtungen. Typischerweise kann eine die verschiedenen Komponenten des Liposoms einschließlich 1 bis 50 Mol-% einer Verbindung der Formel I enthaltende Lösung in eine Lipidsuspension überführt werden, die dann unter hohem Druck durch Düsen oder durch Lochscheiben gepresst wird, wobei durch die Größe der Öffnungen in der Lochscheibe die Größe der erhaltenen Liposomen geregelt werden kann. Geeignete Maßnahmen zur Überführung einer Lipidsuspension in Liposomen sind dem Fachmann bekannt. Bevorzugt werden 5 bis 55 Mol-% einer Verbindung der allgemeinen Formel I mit 35 bis 60 Mol-% Cholesterin und 40 bis 60 Mol-% Phospholipiden oder/und Alkylphospholipiden in eine Lipidsuspension überführt, die dann durch geeignete Maßnahmen auf an sich bekannte Weise in Liposomen umgewandelt wird. Solch bekannte Verfahren können auch zur Herstellung einer pharmazeutischen Zubereitung, die die erfindungsgemäßen Liposomen und einen oder mehrere pharmazeutische Wirkstoffe enthält, verwendet werden. Zum Einschluss wasserunlöslicher Wirkstoffe wird der Wirkstoff dabei zusammen mit den Lipidbestandteilen gelöst, während zum Einschluss wasserlöslicher Wirkstoffe der Lipidfilm mit einer wässrigen Lösung versetzt wird, die den wasserlöslichen Wirkstoff enthält.

Die Ausgangsphospholipide werden nach in der Literatur beschriebenen Verfahren hergestellt (DE 31 30 867 A1; Eibl et al., Chem. Phys. Lipids 28 (1981), 1-5, 41 (1986), 53-63 und 47 (1988), 47-53. Insbesondere kann hier auf Verfahren zurückgegriffen werden, die in der PCT/EP97/O0749-Anmeldung vom 17.02.1997 beschrieben worden sind. So können die erfindungsgemäßen Verbindungen der Formel I auf folgende Weise hergestellt werden:

### Beispiel (A = a; n = 2; y = 1; z = 2)

### (Reaktion von 1,2-Diplamitoyl-sn-glycero-3-phospho-(N-methyl)-ethanolamin mit 1,2-lsopropyliden-glycero-glycidol)

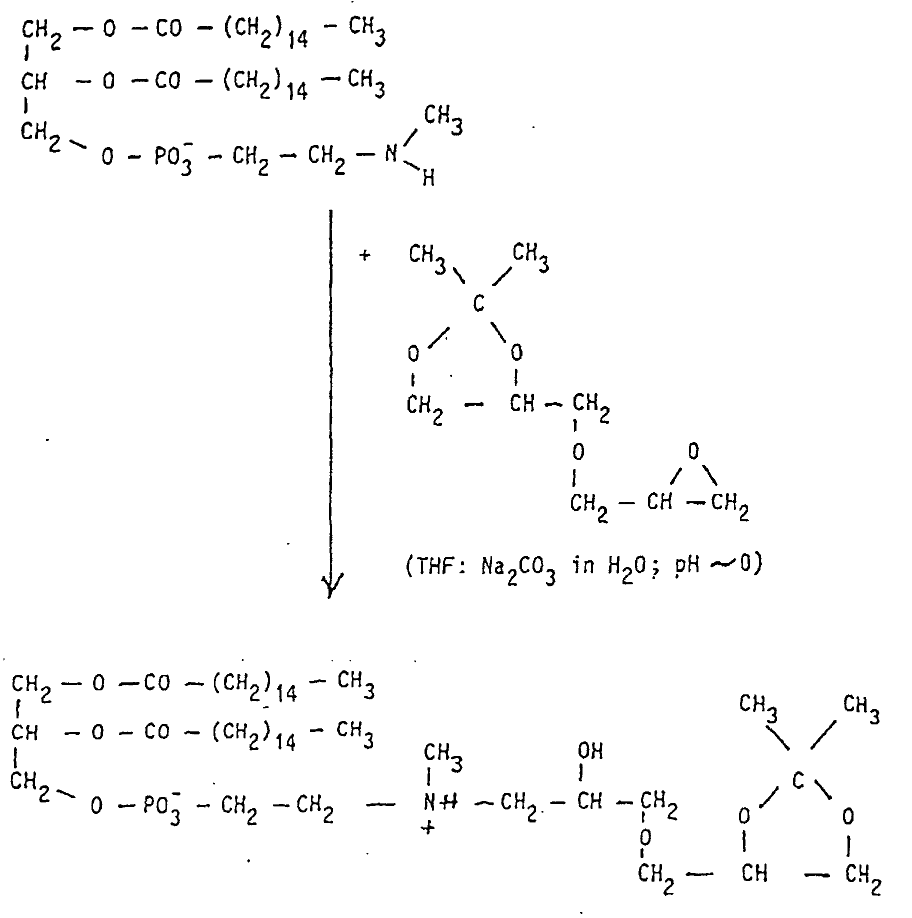

Entsprechende Umsetzungen können durchgeführt werden, die Verbindungen mit n = 3 - 10; y = 1; z = 1 - 5. Ausgangsverbindungen für x = 2 und 3 sind Kephaline und N-Methylkephaline, deren Darstellung ausführlich beschrieben wurde. Die Umsetzungen mit den entsprechenden Glycidolen, deren Herstellung in der deutschen Patentanmeldung "Phosphatidyloligoglycerine" 19622224 beschrieben ist, führt zu den gewünschten Produkten, wobei die Addition in einem 2-Phasensystem THF - Na₂CO₃/NaHCO₃ 1 : 1 (0,5 M in H₂O; pH 9 - 10) durchgeführt wird. Bei diesen pH-Werten wird jedoch keine Hydrolyse der Fettsäureester beobachtet.

Zur Darstellung der Dioleoylverbindungen muss 1,2-Dioleyol-sn-glycero-3-phospho-(N-methyl)-ethanolamin hergestellt werden. Dies wird durch folgendes Reaktionsschema erreicht:

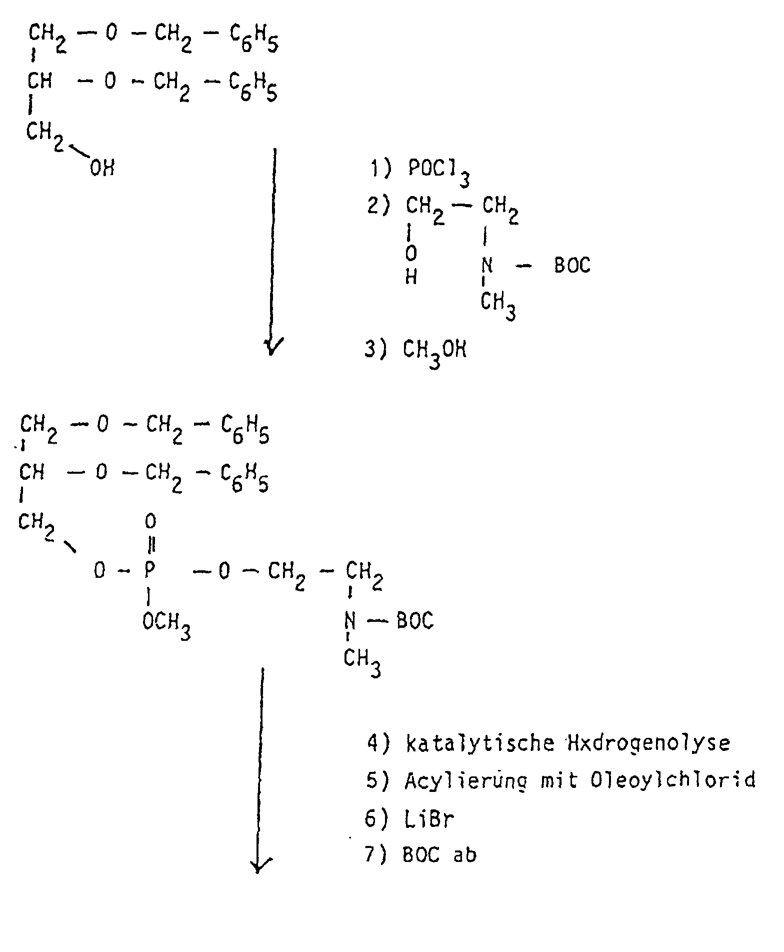

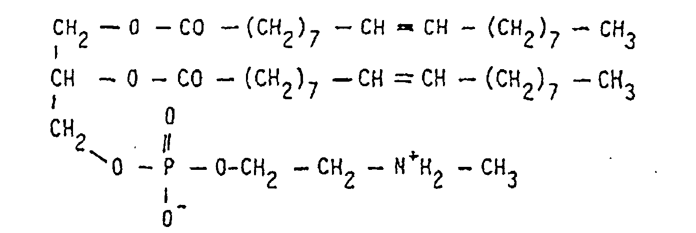

Analog werden allgemein ungesättigte Verbindungen hergestellt.

Entsprechend können Verbindungen mit n = 4 - 8 hergestellt werden, da die entsprechenden endständigen Alkanolamine käuflich erworben und in die N-BOC geschützten Verbindungen umgewandelt werden können.

In der Folge werden Beispiele zur Synthese von Liposomenbestandteilen beschrieben, die die experimentelle Breite der vorliegenden Anmeldung belegen. Es können, wie aus den Beispielen hervorgeht, beliebige Fettsäureester- und Alkyletherkombinationen hergestellt werden, die in Kettenlänge, Anzahl der cis-Doppelbindungen und Verzweigungsgrad variieren.

Die RF-Werte der Beispielsverbindungen 1 bis 279 wurden im System CHCl₃/CH₃OH (Eisessig/H₂O: 100/60/20/5 Volumentanteile bestimmt. Sie liegen gruppenweise sehr dicht beisammen und zwar wie folgt:

| RF | Verbindung Nr.: |
|---|---|
| 0,10-0,15 | 129-135 |
| 0,15-0,20 | 117-128; 167,172;209-216 |
| 0,20-0,25 | 70-84; 98-116; 151-166; 197-208 |
| 0,25-0,30 | 45-69; 136-150; 183-196; 262-272 |
| 0,30-0,35 | 25-44; 173-182; 255-261; 273-279 |
| 0,35-0,40 | 1-24 |
| 0,40-0,45 | 85-97 |
| 0,30-0,40 | 217-240 |
| 0,20-0,30 | 241-254 |

### BEISPIELE

### A) Herstellung von Erucoyl-propandiol-(1,3)-phopsho-N,N,N-trimethylpropylammonium

### (Beispiel 176)

### Erucoyl-propandiol-(1,3)

Propandiol-(1,3), 153 g (MG 76,1; 2 Mol) wird in 1 I THF gelöst, mit 60 g Triethylamin (MG 101,2; 0,6 Mol) und 7,3 g 4-Dimethylaminopyridin (MG 122,2; 0,06 Mol) versetzt und in einem Wasserbad auf 20°C temperiert. Unter stetem Rühren werden 178 g Erucoylchlorid (MG 35,0; 0,5 Mol) in 500 ml THF langsam so zugetropft, daß die Temperatur 30°C im Reaktionsgemisch nicht übersteigt. Nach dem Eintropfen wird noch 30 Minuten auf 30°C erwärmt, mit 1,5 I Diisopropylether und 1,5 I 1N HCl versetzt. Nach gutem Schütteln und Phasenseparation wird die obere Etherphase nochmals mit 1 % NaCl-Lösung gewaschen und in Vakuum bei 45°C einrotiert. Der Rückstand wird in 2 I Hexan aufgenommen und auf -20°C gekühlt. Die weißen Kristalle werden abgesaugt und im Vakuum getrocknet. Die Ausbeute an reinem Erucoyl-propandiol-(1,3) beträgt 123 g (MG 396,7; 62 %). Die Substanz ist dünnschichtchromatographisch rein (Rf-Wert 0,3 in Ether/Petnan/Essigsäure 200/200/2; Volumenanteile).

### Mikroanalyse

| | | | | |
|---|---|---|---|---|
| C₂₅H₄₈O₃ | - Ber. | C, 75,70; | H, 12,20; | O, 12,10 |
| | - Gef. | C, 75,81; | H, 12,16; | =; - |

### Erucoyl-propandiol-(1,3)-phospho-N-methyl-propylammonium

Phophoroxychlorid, 24,2 g (MG 153,33; 0,16 Mol) in 15 ml THF wird in einem Eisbad auf 0°C gekühlt. Man versetzt tropfenweise unter Rühren mit einer Lösung von Erucoyl-propandiol-(1,3), 60 g (MG 396,7; 0,15 Mol) und 17,2 g Triethylamin (MG 101,19; 0,17 Mol) in 250 ml THF, so daß die Temperatur im Reaktionsgemisch 15°C nicht übersteigt. Nach dem Eintropfen der Lösung wird die Temperatur des Reaktionsgemisches auf 20°C gebracht und 30 Minuten weitergerührt.

Die Umwandlung des gebildeten Erucoyl-propandiol-(1,3)-phosphorsäuredichlorids in das Zielprodukt erfolgt durch Umsetzung mit N-Methylpropanolamin über einen intermediären Sechsring. Dazu wird das Reaktionsgemisch unter Rühren mit einer Lösung von 16 g N-Methylpropanolamin (MG 89,14; 0,18 Mol) und 35,4 g Triethylamin (MG 101, 19; 035 Mol) in 250 ml THF tropfenweise so versetzt, daß die Temperatur 35°C nicht übersteigt. Nach dem Eintropfen wird das Reaktionsgemisch noch 30 Minuten bei 25°C gehalten. Das ausgefallene Triethylaminhydrochloird wird abfiltriert. Das Filtrat enthält das Zwischenprodukt (Rf 0,25 in CHCl₃/Essigester 1:1; Volumenanteile), das durch Zusatz von 60 ml 2 N HCI unter Ringöffnung in Erucoyl-propandiol-(1,3)-phospho-N-methyl-propylammonium (Rf 0,45 in CHCl₃/CH₃OH/Eisessig/H₂O 100/80/10/5, Volumenteile)umgewandelt wird. Die THF-Lösung des Produkts wird mit 200 ml 0,2 M Natriumphosphatlösung (pH ∼ 8,0) versetzt und auf pH 6 - 7 gebracht. Man fällt das Produkt durch Zugabe von 1 I Aceton aus und isoliert die Kristalle durch Absaugen bei 4°C. Erucoyl-propandiol-(1,3)-phospho-N-methyl-propylammonium ist manchmal leicht verunreinigt. Zur Reinigung kann eine Cromatographie an Kieselgel mit CHCl₃/CH₃OH/H₂O eingesetzt werden. Die Ausbeute an reinem Produkt - bezogen auf Erucoyl-propandiol-(1,3) - beträgt 65 g (MG 547,8; 79 %).

### Mikroanalyse

| | | | | | | |
|---|---|---|---|---|---|---|
| C₂₉H₅₈NO₆P | - Ber. | C, 63,59; | H, 10,67; | N, 2,56; | O, 17,53; | P, 5,66 |
| | - Gef. | C, 63,34; | H, 10,49; | N, 2,49; | O, - ; | P, 5,59 |

### Erucoyl-propandiol-(1,3)-phospho-N,N,N-trimethyl-propylammonium

Erucoyl-propandiol-(1,3)-phospho-N-methyl-propylammonium, 54,8 g (MG 547,8; 0,1 Mol) werdenmit 800 ml THF und 83 g K₂CO₃ (0,6 Mol) in 800 ml H₂O versetzt. Man erwärmt auf 50°C und erhält eine zweiphasige Lösung. Man tropft unter starkem Rühren 74,5 g Toluolsulfonsäuremethylester (MG 186,23; 0,4 Mol) in 200 ml THF ein und kocht unter Rückfluß. Die Reaktion ist nach 60 Minuten beendet. Erucoylpropandiol-(1,3)-phospho-N,N,N-trimethyl-propylammonium als Produkt (Rf-Wert 0,1 in CHCl₃/CH₃OH/Eisessig/H₂O 100/80/10/5, Volumenanteile) wird aus der THF-Phase mit 1,2 1 Aceton ausgefällt, der Niederschlag in 300 ml CHCl₃ aufgenommen, filtriert und nochmals mit 1,2 I Aceton gefällt. Ist das Produkt nicht ganz rein, kann zur Aufreinigung eine Chromatographie an Kieselgel mit CHCl₃/CH₃OH/H₂O eingesetzt werden. Die Ausbeute an reinem Produkt - bezogen auf die N-methyl-Verbindung - beträgt 49 g (MG 575,8M 85 %).

### Mikroanalyse

| | | | | | | |
|---|---|---|---|---|---|---|
| C₃₁H₆₂NO₆P | - Ber. | C, 64,66; | H, 10,85; | N, 2,43; | O, 16,67; | P, 5,38 |
| | - Gef. | C, 64,38; | H, 10,81; | N, 2,39; | O, -; | P, 5,27 |

### B) Herstellung von Erucoyl-propandiol-(1,3)-phospho-N,N-dimethyl-N-(hydroxypropyl-dihydroxypropyl)-propylammonium

### (Beispiel 155)

Zur Herstellung dieser Verbindung kann das Zwischenprodukt Erucoylpropandiol-(1,3)-phospho-N-methyl-propylammoniumeingesetztwerden.Es wird in einer Reaktion zuerst mit einem Epoxyd umgesetzt und direkt weiter zum Endprodukt methyliert.
Erucoyl-propandiol-(1,3)-phospho-N-methyl-propylammonium, 54,8 g (MG 547,8; 0,1 Mol) werdenmit 800 ml THF un d83 g K₂CO₃ (0,6 Mol) in 800 ml H₂O versetzt, man erwärmt auf 50°C und erhält eine zweiphasige Lösung. Man tropft unter starkem Rühren eine Lösung aus 21 g 1,2-lsopropyliden-glycero-3,1-glycidol (MW 188,2; 0,11 Mol) in 200 ml THF ein und erhöht die Temperatur auf 60°C. Das Reaktionsgemisch wird 2 Stunden unter leichtem Rückfluß gekocht und mit 37 g Toluolsulfonsäuremethylester (MG 186,23; 0,2 Mol) in 100 ml THF versetzt. Nach 2 Stunden Kochen unter Rückfluß ist die Reaktion beendet. Die THF-Phase wird bei 45°C weitgehend einrotiert und der Rückstand zur Entfernung der Isopropylidenschutzgruppe mit 300 ml 70 % Essigsäure auf 60 - 65°C erhitzt. Man versetzt das Reaktionsgemisch mit einem Gemisch aus 1 I CHCl₃/1 I CH₃OH/1 I 1 %ige NaCI-Lösung, schüttelt gut durch und entfernt das Lösungsmittel aus der unteren CHCl₃-Phase im Vakuum. Der Rückstand wird in 400 ml CHCl₃ aufgenommen und mit 1,6 I Aceton gefällt. Zur Reinigung kann eine Chromatographie an Kieselgel mit CHCl₃/CH₃OH/H₂O eingesetzt werden. Man erhält 46 g Erucoyl-propandiol-(1,3)-phospho-N,N-dimethyl-N-(hydroxypropyl-3,1-dihydroxypropyl)-propylammonium (MG 709,94; 65 %).

Alle nachstehend aufgeführten Verbindungen können nach diesen allgemeinen Vorschriften zur Herstellung sowohl am Stickstoff hydroxylierter wie auch nicht hydroxylierter Verbindungen dargestellt werden.

### Beispiele für zweikettige Glycero-phospho-N,N-dimethyl-N-dihydroxypropylalkylammonium-Verbindungen

(A = a; n = 2-6; R₃, CH₃; x = 0; y = 1; z = 1) 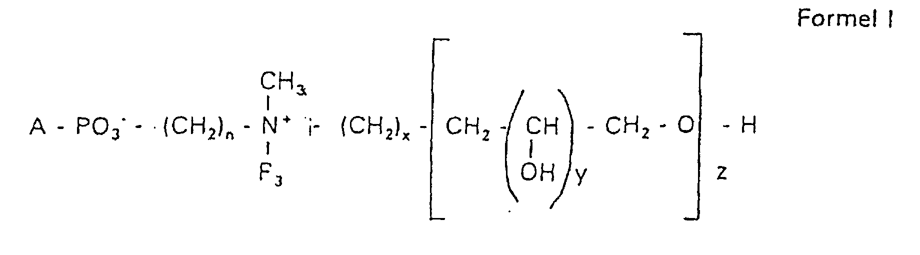
1) 1,2-Dipalmitoyl-sn-glycero-3-phospho-N,N-dimethyl-N-dihydroxypropyl-ethylammonium (n = 2)
   (R₁, R₂ = CO - (CH₂)₁₄ - CH₃)
   C₄₂H₈₄NO₁₀P (794,10)
2) 1,2-Distearoyl-sn-glycero-3-phospho-N,N-dimethyl-N-dihydroxypropylethylammonium (n = 2)
   (R₁, R₂ = CO - (CH₂)₁₆ - CH₃)
   C₄₆H₉₂NO₁₀P (850,20)
3) 1,2-Dimyristoyl-sn-glycero-3-phospho-N,N-dimethyl-N-dihydroxypropyl-ethylammonium (n = 2)
   (R₁, R₂ = CO - (CH₂)₁₂ - CH₃)
   C₃₈H₇₆NO₁₀P (737,99)
4) 1,2-Dierucyl-sn-glycero-3-phospho-N,N-dimethyl-N-dihydroxypropylethylammonium (n = 2)
   (R₁, R₂ = CO - (CH₂)₁₁ - CH = CH - (CH₂)₇ - CH₃)
   C₅₄H₁₀₄NO₁₀P (958,39)
5) 1,2-Dioleoyl-sn-glycero-3-phospho-N,N-dimethyl-N-dihydroxypropylethylammonium (n = 2)
   (R₁, R₂ = CO - (CH₂)₇ - CH = CH - (CH₂)₇ - CH₃)
   C₄₆H₈₈NO₁₀P (846,17)
6) 1-Stearoyl-2-oleoyl-sn-glycero-3-phospho-N,N-dimethyl-N-dihydroxypropyl-ethylammonium (n = 2)
   (R₁ = CO-(CH₂)₁₆ - CH₃; R₂ = CO - (CH₂)₁₇ - CH = CH - (CH₂)₇-CH₃)
   C₄₆H₉₀NO₁₀P (848,19)
7) 1-Stearoyl-2-myristoyl-sn-glycero-3-phospho-N,N-dimethyl-N-dihydroxypropyl-ethylammonium (n = 2)
   (R₁ = CO - (CH₂)₁₆ - CH₃; R₂ = CO - (CH₂)₁₂ - CH₃)
   C₄₂H₈₄NO₁₀P (794,10)
8) 1-Stearoyl-2-lauroyl-sn-glycero-3-phospho-N,N-dimethyl-N-dihydroxypropyl-ethylammonium (n = 2)
   (R₁ = CO - (CH₂)₁₆ - CH₃; R₂ = CO - (CH₂)₁₀ - CH₃)
   C₄₀H₈₀NO₁₀P (766,04)
9) 1-Lauroyl-2-stearoyl-sn-glycero-3-phospho-N,N-dimethyl-N-dihydroxypropyl-ethylammonium (n = 2)
   (R₁ = CO - (CH₂)₁₀ - CH₃; R₂ = CO - (CH₂)₁₆ - CH₃)
   C₄₀H₈₀NO₁₀P (766,04)
10) 1-Erucoyl-2-oleoyl-sn-glycero-3-phospho-N,N-dimethyl-N-dihydroxypropyl-ethylammonium (n = 2)
   (R₁ = CO - (CH₂)₁₁ - CH = CH -(CH₂)₇ - CH₃)
   (R₂ = CO - (CH₂)₇ - CH = CH - (CH₂)₇ - CH₃)
   C₅₀H₉₆NO₁₀P (902,28)
11) 1-Oleoyl-2-erucyl-sn-glycero-3-phospho-N,N-dimethyl-N-dihydroxypropyl-ethylammonium (n = 2)
   (R₁ = CO - (CH₂)₇ - CH = CH - (CH₂)₇ - CH₃)
   (R₂ = CO - (CH₂)₁₁ - CH = CH - (CH₂)₇ - CH₃)
   C₅₀H₉₆NO₁₀P (902,28)
12) 1,2-Dipalmitoyl-sn-glycero-3-phospho-N,N-dimethyl-N-dihydroxypropyl-ethylammonium (n = 3)
   (R₁, R₂ = Cl - (CH₂)₁₄ - CH₃)
   C₄₃H₈₆NO₁₀P (808,12)
13) 1,2-Dierucyl-sn-glycero-3-phospho-N,N-dimethyl-N-dihydroxypropylethylammonium (n = 3)
   (R₁, R₂ = CO - (CH₂)₁₁ - CH = CH - (CH₂)₇ - CH₃)
   C₅₅H₁₀₆NO₁₀P (972,413)
14) 1,2-Dioleoyl-sn-glycero-3-phospho-N,N-dimethyl-N-dihydroxypropylethylammonium (n = 3)
   (R₁, R₂ = CO - (CH₂)₇ - CH = CH - (CH₂)₇ - CH₃)
   C₄₇H₉₀NO₁₀P (860,20)
15) 1-Stearoyl-2-oleoyl-sn-glycero-3-phospho-N,N-dimethyl-N-dihydroxypropyl-ethylammonium (n = 3)
   (R₁ = CO - (CH₂)₁₆ - CH₃; R₂ = CO - (CH₂)₇ - CH = CH - (CH₂)₇ - CH₃)
   C₄₇H₉₂NO₁₀P (862,21)
16) 1-Stearoyl-2-lauroyl-sn-glycero-3-phospho-N,N-dimethyl-N-dihydroxypropyl-ethylammonium (n = 3)
   (R₁ = CO - (CH₂)₁₆ - CH₃; R₂ = CO - (CH₂)₁₀ - CH₃)
   C₄₁H₈₂NO₁₀P (780,07)
17) 1,2-Dipalmitoyl-sn-glycero-3-phospho-N,N-dimethyl-N-dihydroxypropyl-butylammonium (n = 4)
   (R₁, R₂ = CO - (CH₂)₁₄ - CH₃)
   C₄₄H₈₈NO₁₀P (822,15)
18) 1,2-Dioleoyl-sn-glycero-3-phospho-N,N-dimethyl-N-dihydroxypropylbutylammonium (n = 4)
   (R₁, R₂ = CO - (CH₂)₇ - CH = CH - (CH₂)₇ - CH₃)
   C₄₈H₉₂NO₁₀P (874,23)
19) 1,2-Dierucyl-sn-glycero-3-phospho-N,N-dimethyl-N-dihydroxypropylbutylammonium (n = 4)
   (R₁, R₂ = CO - (CH₂)₇ - CH = CH - (CH₂)₇ - CH₃)
   C₅₆H₁₀₈NO₁₀P (986,44)
20) 1-Stearoyl-2-oleoyl-sn-glycero-3-phospho-N,N-dimethyl-N-dihydroxypropyl-butylammonium (n = 4)
   (R₁ = CO - (CH₂)₁₆ - CH₃; R₂ = CO - (CH₂)₇ - CH = CH - (CH₂)₇ - CH3)
   C₄₈H₉₄NO₁₀P (876,24)
21) 1-Stearoyl-2-lauroyl-sn-glycero-3-phospho-N,N-dimethyl-N-dihydroxypropyl-butylammonium (n = 4)
   (R₁ = CO - (CH₂)₁₆ - CH₃; R₂ = CO - (CH₂)₁₀ - CH₃)
   C₄₂H₈₄NO₁₀P (794,10)
22) 1,2-Dipalmitoyl-sn-glycero-3-phospho-N,N-dimethyl-N-dihydroxypropyl-hexylammonium (n = 6)
   (R₁, R₂ = CO - (CH₂)₁₄ - CH₃)
   C₄₂H₈₄NO₁₀P (794,10)
23) 1,2-Dioleoyl-sn-glycero-3-phospho-N,N-dimethyl-N-dihydroxypropylhexylammonium (n = 6)
   (R₁, R₂ = CO - (CH₂)₇ - CH = CH - (CH₂)₇ - CH₃)
   C₅₀H₉₆NO₁₀P (902,28)
24) 1,2-Dierucyl-sn-glycero-3-phospho-N,N-dimethyl-N-dihydroxypropylhexylammonium (n = 6)
   (R₁, R₂ = CO - (CH₂)₁₁ - CH = CH - (CH₂)₇ - CH₃)
   C₅₈H₁₁₂NO₁₀P (1014,49)

### Beispiele für zweikettige Glycero-phospho-N,N-dimethyl-N-(2-hydroxypropyl-3,1-O,O-dihydroxypropyl)-alkylammonium-Verbindungen

(A = a; n = 2-6; R₃, O₃; x = 0; y = 1; z = 2) 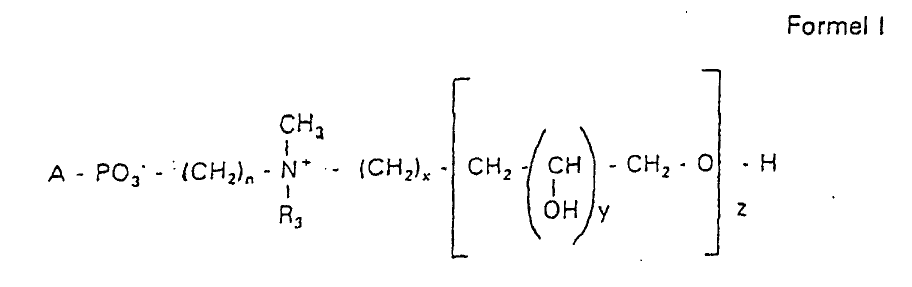
25) 1,2-Dipalmitoyl-sn-glycero-3-phospho-N,N-dimethyl-N-(2-hydroxypropyl-3,1-0,0-dihydroxypropyl)-ethylammonium (n = 2)
   (R₁; R₂ = CO - (CH₂)₁₄ - CH₃)
   C₄₅H₉₀NO₁₂P (868,18)
26) 1,2-Distearoyl-sn-glycero-3-phospho-N,N-dimethyl-N-(2-hydroxypropyl-3,1-0,0-dihydroxypropyl)ethylammonium (n = 2)
   (R₁, R₂ = CO - (CH₂)₁₆ - CH₃)
   C₄₉H₉₈NO₁₂P (924,28)
27) 1,2-Dierucoyl-sn-glycero-3-phospho-N,N-dimethyl-N-(2-hydroxypropyl-3,1-0,0-dihydroxypropyl)-ethylammonium (n = 2)
   (R₁, R₂ = CO - (CH₂)₁₁ - CH = CH - (CH₂)₇ - CH₃)
   C₅₇H₁₁₀NO₁₂P (1032,47)
28) 1,2-Dioleoyl-sn-glycero-3-phospho-N,N-dimethyl-N-(2-hydroxypropyl-3,1-0,0-dihydroxypropyl)-ethylammonium (n = 2)
   (R₁, R₂ = CO - (CH₂)₇ - CH = CH - (CH₂)₇ - CH₃)
   C₄₉H₉₄NO₁₂P (920,25)
29) 1-Stearoyl-2-oleoyl-sn-glycero-3-phospho-N,N-dimethyl-N-(2-hydroxypropyl-3,1-O,O-dihydroxypropyl)-ethylammonium (n = 2)
   (R₁ = CO - (CH₂)₁₆ - CH₃; R₂ = CO - (CH₂)₇ - CH = CH - (CH₂)₇ - CH₃)
   C₄₉H₉₆NO₁₂P (922,27)
30) 1-Stearoyl-2-lauroyl-sn-glycero-3-phospho-N,N-dimethyl-N-(2-hydroxypropyl-3,1-O,O-dihydroxypropyl)-ethylammonium (n = 2)
   (R₁ = CO - (CH₂)₁₆ - CH₃; R₂ = CO - (CH₂)₁₀ - CH₃)
   C₄₃H₈₆NO₁₂P (840,12)
31) 1-Erucoyl-2-oleoyl-sn-glycero-3-phospho-N,N-dimethyl-N-(2-hydroxypropyl-3,1-O,O-dihydroxypropyl)-ethylammonium (n = 2)
   (R₁ = CO - (CH₂)₁₁-CH = CH - (CH₂)₇ - CH₃;
   R₂ = CO - (CH₂)₇ - CH = CH - (CH₂)₇ - CH₃)
   C₅₃H₁₀₂NO₁₂P (976,36)
32) 1-Oleoyl-2-erucoyl-sn-glycero-3-phospho-N,N-dimethyl-N-(2-hydroxypropyl-3,1-O,O-dihydroxypropyl)-ethylammonium (n = 2)
   (R₁ = CO - (CH₂)₇ - CH = CH - (CH₂)₇ - CH₃;
   R₂ = CO - (CH₂)₁₁ - CH = CH - (CH₂)₇ - CH₃)
   C₅₃H₁₀₂NO₁₂P (976,36)
33) 1,2-Dipalmitoyl-sn-glycero-3-phospho-N,N-dimethyl-N-(2-hydroxypropyl-3,1-O,O-dihydroxypropyl)-propylammonium (n = 3)
   (R₁, R₂ = CO - (CH₂)₁₄ - CH₃)
   C₄₆H₉₂NO₁₂P (882,20)
34) 1,2-Distearoyl-sn-glycero-3-phospho-N,N-dimethyl-N-(2-hydroxypropyl-3,1-0,0-dihydroxypropyl)-propylammonium (n = 3)
   (R₁, R₂ = CO - (CH₂)₁₆ - CH₃)
   C₅₀H₁₀₀NO₁₂P (938,31)
35) 1,2-Dierucoyl-sn-glycero-3-phospho-N,N-dimethyl-N-(2-hydroxypropyl-3,1-O,O-dihydroxypropyl)-propylammonium (n = 3)
   (R₁, R₂ = CO - (CH₂)₁₁ - CH = CH - (CH₂)₇ - CH₃)
   C₅₈H₁₁₂NO₁₂P (1046,49)
36) 1,2-Dioleoyl-sn-glycero-3-phospho-N, N-dimethyl-N-(2-hydroxypropyl-3,1-O,O-dihydroxypropyl)-propylammonium (n = 3)
   (R₁, R₂ = CO - (CH₂)₇ - CH = CH - (CH₂)₇ - CH₃)
   C₅₀H₉₆NO₁₂P (934,28)
37) 1-Stearoyl-2-oleoyl-sn-glycero-3-phospho-N,N-dimethyl-N-(2-hydroxypropyl-3,1-O,O-dihydroxypropyl)propylammonium(n = 3)
   (R₁ = CO - (CH₂)₁₆ - CH₃; R₂ = CO - (CH₂)₇ - CH = CH - (CH₂)₇ - CH₃)
   C₅₀H₉₈NO₁₂P (936,29)
38) 1-Stearoyl-2-lauroyl-sn-glycero-3-phospho-N,N-dimethyl-N-(2-hydroxypropyl-3,1-0,0-dihydroxypropyl)-propylammonium (n = 3)
   (R₁ = CO - (CH₂)₁₆ - CH₃; R₂ = CO - (CH₂)₁₀ - CH₃)
   C₄₄H₈₈NO₁₂P (854,15)
39) 1,2-Dioleoyl-sn-glycero-3-phospho-N,N-dimethyl-N-(2-hydroxypropyl-3,1-0,0-dihydroxypropyl)-butylammonium (n = 4)
   (R₁, R₂ = CO - (CH₂)₇ - CH = CH - (CH₂)₇ - CH₃)
   C₅₁H₉₈NO₁₂P (948,30)
40) 1, 2-Dierucoyl-sn-glycero-3-phospho-N,N-dimethyl-N-(2-hydroxypropyl-3,1-O,O-dihydroxypropyl)-butylammonium (n = 4)
   (R₁, R₂ = CO - (CH₂)₁₁ - CH = CH - (CH₂)₇ - CH₃)
   C₅₉H₁₁₄NO₁₂P (1060,52)
41) 1-Stearoyl-2-oleoyl-sn-glycero-3-phospho-N,N-dimethyl-N-(2-hydroxypropyl-3,1-O,O-dihydroxypropyl)-butylammonium (n = 4)
   (R₁ = CO - (CH₂)₁₆ - CH₃; R₂ = CO - (CH₂)₇ - CH = CH - (CH₂)₇ - CH₃)
   C₅₁H₁₀₀NO₁₂P (950,32)
42) 1-Stearoyl-2-lauroyl-sn-glycero-3-phospho-N,N-dimethyl-N-(2-hydroxypropyl-3,1-O,O-dihydroxypropyl)-butylammonium (n = 4)
   (R₁ = CO - (CH₂)₁₆ - CH₃; R₂ = CO - (CH₂)₁₀ - CH₃)
   C₄₅H₉₀NO₁₂P (868,175)
43) 1,2-Dioleoyl-sn-glycero-3-phospho-N,N-dimethyl-N-(2-hydroxypropyl-1,3-0,0-dihydroxypropyl)-hexylammonium (n = 6)
   (R₂, R₂ = CO - (CH₂)₇ - CH = CH - (CH₂)₇ - CH₃)
   C₅₃H₁₀₂NO₁₂P (976,358)
44) 1,2-Dierucoyl-sn-glycero-3-phospho-N,N-dimethyl-N-(2-hydroxypropyl-3,1-O,O-dihydroxypropyl)-hexylammonium (n = 6)
   (R₁, R₂ = CO - (CH₂)₁₁ - CH = CH - (CH₂)₇ - CH₃)
   C₆₁H₁₁₈NO₁₂P (1088,57)

### Beispiele für zweikettige Glycero-phospho-N,N-dimethyl-N-(2-hydroxypropyl-3,1-O,O-2-hydroxypropyl-3,1-O,O-dihydroxypropyl)-alkylammonium-Verbindungen

(A = a; n = 2-8; R₃, CH₃; x = 0; y = 1; z = 3) 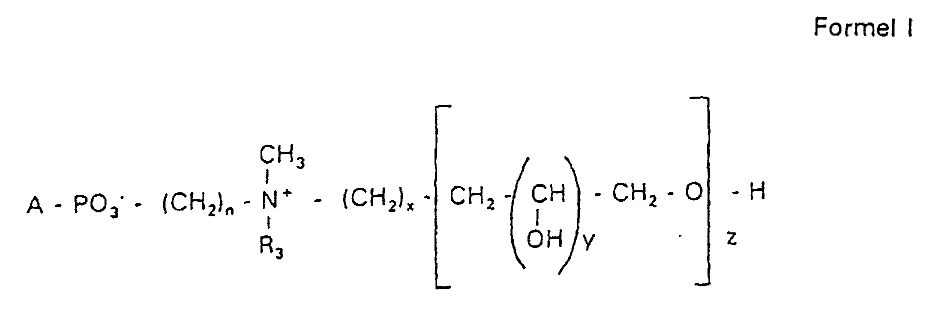
45) 1,2-Dipalmitoyl-sn-glycero-3-phospho-N,N-dimethyl-N-(2-hydroxypropyl-3,1-0,0-2-hydroxypropyl-3,1-dihydroxypropyl)-ethylammonium (n = 2)
   (im weiteren Text wird N-(2-hydroxypropyl-3,1-0,0-2-hydroxypropyl-3,1-dihydroxypropyl) abgekürzt als N-(HP₁-HP₂-diHP₃)
   C₄₈H₉₆NO₁₄P (942,25)
46) 1,2-Distearoyl-sn-glycero-3-phospho-N,N-dimethyl-N-(HP₁HP₂-diHP₃)-ethylammonium (n = 2)
   C₅₂H₁₀₄NO₁₄P (998,36)
47) 1,2-Dierucoyl-sn-glycero-3-phospho-N,N-dimethyl-N-(HP₁-HP₂-diHP₃)-ethylammonium (n = 2)
   C₆₀H₁₁₆NO₁₄P (1106,54)
48) 1,2-Dioleoyl-sn-glycero-3-phospho-N,N-dimethyl-N-(HP₁-HP₂-diHP₃)-ethylammonium ( n = 2)
   C₅₂H₁₀₀NO₁₄P (994,33)
49) 1-Stearoyl-2-oleoyl-sn-glycero-3-phospho-N,N-dimethyl-N(HP₁-HP₂diHP₃)-ethytammonium (n = 2)
   C₅₂H₁₀₂NO₁₄P (996,35)
50) 1-Stearoyl-2-lauroyl-sn-glycero-3-phospho-N,N-dimethyl-N-(HP₁-HP₂diHP₃)-ethylammonium (n = 2)
   C₄₆H₉₂NO₁₄P (914,20)
51) 1-Palmitoyl-2-lauroyl-sn-glycero-3-phospho-N,N-dimethyl-N-(HP₁-HP₂diHP₃)-ethylammonium (n = 2)
   C₄₄H₈₈NO₁₄P (886,15)
52) 1,2-Dipalmitoyl-sn-glycero-3-phospho-N,N-dimethyl-N-(HP₁-HP₂diHP₃)-propylammonium (n = 3)
   C₄₉H₉₈NO₁₄P (956,28)
53) 1,2-Distearoyl-sn-glycero-3-phospho-N,N-dimethyl-N-(HP₁-HP₂-diHP₃)-propylammonium (n = 3)
   C₅₃H₁₀₆NO₁₄P (1012,39)
54) 1,2-Dierucoyl-sn-glycero-3-phospho-N,N-dimethyl-N-(HP₁-HP₂-diHP₃)-propylammonium (n = 3)
   C₆₁H₁₁₈NO₁₄P (1120,57)
55) 1,2-Dioleoyl-sn-glycero-3-phospho-N,N-dimethyl-N-(HP₁-HP₂-diHP₃)-propylammonium (n = 3)
   C₅₃H₁₀₂NO₁₄P (1008,36)
56) 1-Stearoyl-2-oleoyl-sn-glycero-3-phospho-N,N-dimethyl-N-(HP₁-HP₂diHP₃)-propylammonium (n = 3)
   C₅₃H₁₀₄NO₁₄P (1010,37)
57) 1-Stearoyl-2-lauroyl-sn-glycero-3-phospho-N,N-dimethyl-N-(HP₁-HP₂diHP₃)-propylammonium (n = 3)
   C₄₇H₉₄NO₁₄P (928,23)
58) 1-Palmitoyl-2-lauroyl-sn-glycero-3-phospho-N,N-dimethyl-N-(HP₁-HP₂diHP₃)-propylammonium (n = 3)
   C₄₅H₉₀NO₁₄P (900,17)
59) 1,2-Dipalmitoyl-sn-glycero-3-phospho-N,N-dimethyl-N-(HP₁-HP₂diHP₃)-butylammonium (n = 4)
   C₅₀H₁₀₀NO₁₄P (970,31)
60) 1,2-Distearoyl-sn-glycero-3-phospho-N,N-dimethyl-N-(HP₁-HP₂-diHP₃)-butylammonium (n = 4)
   C₅₄H₁₀₈NO₁₄P (1026,41)
61) 1,2-Dierucoyl-sn-glycero-3-phospho-N,N-dimethyl-N-(HP₁-HP₂-diHP₃)-butylammonium (n = 4)
   C₆₂H₁₂₀NO₁₄P (1134,60)
62) 1,2-Dioleoyl-sn-glycero-3-phospho-N,N-dimethyl-N-(HP₁-HP₂-diHP₃)-butylammonium (n = 4)
   C₅₄H₁₀₄NO₁₄P (1022,38)
63) 1,2-Dipalmitoyl-sn-glycero-3-phospho-N,N-dimethyl-N-(HP₁-HP₂diHP₃)-hexylammonium (n = 6)
   C₅₂H₁₀₄NO₁₄P (998,36)
64) 1,2-Distearoyl-sn-glycero-3-phospho-N,N-dimethyl-N-(HP₁-HP₂-diHP₃)-hexylammonium (n = 6)
   C₅₆H₁₁₂NOP₁₄P (1054,47)
65) 1,2-Dierucoyl-sn-glycero-3-phospho-N,N-dimethyl-N-(HP₁-HP₂-diHP₃)-hexylammonium (n = 6)
   C₆₄H₁₂₄NO₁₄P (1162,65)
66) 1,2-Dioleoyl-sn-glycero-3-phospho-N,N-dimethyl-N-(HP₁-HP₂-diHP₃)-hexylammonium in = 6)
   C₅₆H₁₀₈NO₁₄P (1050,44)
67) 1,2-Distearoyl-sn-glycero-3-phospho-N,N-dimethyl-N-(HP₁-HP₂-diHP₃)-octylammonium (n = 8)
   C₅₈H₁₁₆NO₁₄P (1082,52)
68) 1,2-Dioleoyl-sn-glycero-3-phospho-N,N-dimethyl-N-(HP₁-HP₂-diHP₃)-octylammonium (n = 8)
   C₅₈H₁₁₂NO₁₄P (1078,49)
69) 1,2-Dierucoyl-sn-glycero-3-phospho-N,N-dimethyl-N-(HP₁-HP₂-diHP₃)-octylammonium (n = 8)
   C₆₆H₁₂₈NO₁₄P (1190,70)

### Beispiele für zweikettige Glycero-phospho-N,N-dimethyl-N-(2-hydroxypropyl-3,1-O,O-2-hydroxypropyl-3,1-O,O-2-hydroxypropyl-3,1-O,O-dihydroxypropyl)-alkylammonium-Verbindungen

(A = a; n = 2, 3; R₃, O₃; x = 0; y = 1; z = 4) 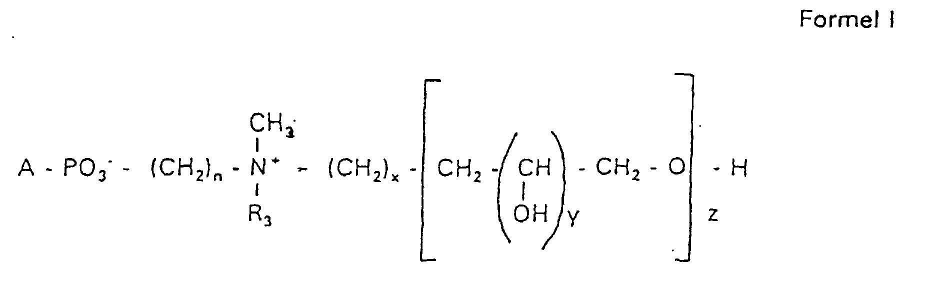
70) 1,2-Dipalmitoyl-sn-glycero-3-phospho-N,N-dimethyl-N-(2-hydroxypropyl-3,1-O,O-2-hydroxypropyl-3,1-O,O-hydroxypropyl-3,1-dihydroxypropyl)-ethylammonium (n = 2)
   (im weiteren Text wird N[2-hydroxypropyl-3,1-O,O-2-hydroxypropyl-3,1-O,O-dihydroxypropyl-3,1-0,0-dihydroxypropyl] abgekürzt als N-(HP₁-HP₂-HP₃-diHP₄) angegeben.
   C₅₁H₁₀₂NO₁₆P (1016,33)
71) 1,2-Distearoyl-sn-glycero-3-phospho-N,N-dimethyl-N-(HP₁-HP₂-HP₃diHP₄)-ethylammonium (n = 2)
   C₅₅H₁₁₀NO₁₆P (1072,44)
72) 1,2-Dierucoyl-sn-glycero-3-phospho-N,N-dimethyl-N-(HP₁-HP₂-HP₃diHP₄)-ethylammonium (n = 2)
   C₆₃H₁₂₂NO₁₆P (1180,62)
73) 1,2-Dioleoyl-sn-glycero-3-phospho-N,N-dimethyl-N-(HP₁-HP₂-HP₃diHP₄)-ethylammonium (n = 2)
   C₅₅H₁₀₆NO₁₆P (1068,41)
74) 1-Stearoyl-2-oleoyl-sn-glycero-3-phospho-N,N-dimethyl-N-(HP₁-HP₂-HP₃-diHP₄)-ethylammonium (n = 2)
   C₅₅H₁₀₈NO₁₆P (1070,42)
75) 1-Stearoyl-2-lauroyl-sn-glycero-3-phospho-N,N-dimethyl-N-(HP₁-HP₂-HP₃-diHP₄)-ethylammonium (n = 2)
   C₄₉H₉₈NO₁₆P (988,28)
76) 1-Palmitoyl-2-lauroyl-sn-glycero-3-phospho-N,N-dimethyl-N-(HP₁-HP₂-HP₃-diHP₄)-ethylammonium (n = 2)
   C₄₇H₉₄NO₁₆P (960,23)
77) 1,2-Dipalmitoyl-sn-glycero-3-phospho-N,N-dimethyl-N-(HP₁-HP₂-HP₃diHP₄)-propylammonium (n = 3)
   C₅₂H₁₀₄NO₁₆P (1030,36)
78) 1,2-Distearoyl-sn-glycero-3-phospho-N,N-dimethyl-N-(HP₁-HP₂-HP₃diHP₄)-propylammonium (n = 3)
   C₅₆H₁₁₂NO₁₆P (1086,47)
79) 1,2-Dierucoyl-sn-glycero-3-phospho-N,N-dimethyl-N-(HP₁-HP₂-HP₃diHP₄)-propylammonium (n = 3)
80) 1,2-Dioleoyl-sn-glycero-3-phospho-N,N-dimethyl-N-(HP₁-HP₂-HP₃diHP₄)-propylammonium (n = 3)
   C₅₆H₁₀₈NO₁₆P (1082,43)
81) 1-Stearoyl-2-lauroyl-sn-glycero-3-phospho-N,N-dimethyl-N-(HP₁-HP₂-HP₃-diHP₄)-propylammonium (n = 3)
   C₅₀H₁₀₀NO₁₆P (1002,31)
82) 1-Stearoyl-2-oleoyl-sn-glycero-3-phospho-N,N-dimethyl-N-(HP₁-HP₂-HP₃-diHP₄)-propylammonium (n = 3)
   C₅₆H₁₁₀NO₁₆P (1084,45)
83) 1-Arachinoyl-2-oleoyl-sn-glycero-3-phospho-N,N-dimethyl-N-(HP₁-HP₂-HP₃-diHP₄)-propylammonium (n = 3)
   C₅₈H₁₁₄NO₁₆P (1112,50)
84) 1-Behenoyl-2-oleoyl-sn-glycero-3-phospho-N,N-dimethyl-N-(HP₁-HP₂-HP₃-diHP₄)-propylammonium (n = 3)
   C₆₀H₁₁₈NO₁₆P (1140,56)

### Beispiele für zweikettige Glycero-phospho-Verbindungen, die nicht am Stickstoff hydroxyliert sind

(A = a; n = 2-6; x = 1; z = 0) 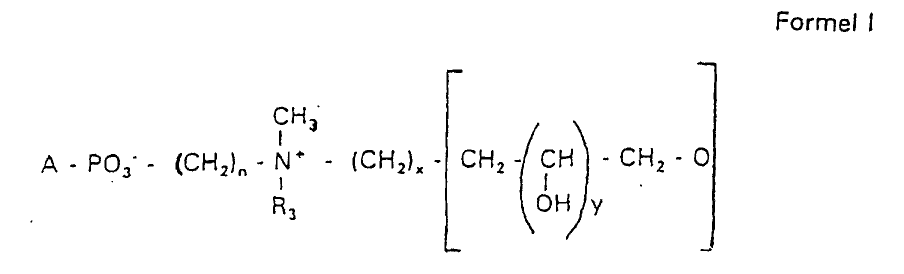
85) 1,2-Dioleoyl-sn-glycero-3-phospho-N,N,N-trimethyl-propylammonium
   C₄₅H₈₆NO₈P (800,15)
86) 1,2-Dioleoyl-sn-glycero-3-phospho-N,N,N-trimethyl-butylammoniüm
   C₄₆H₈₈NO₈P (814,17)
87) 1,2-Dioleoyl-sn-glycero-3-phospho-N,N,N-trimethyl-pentylammonium
   C₄₇H₉₀NO_{P} (828,20)
88) 1,2-Dioleoyl-sn-glycero-3-phospho-N,N,N-trimethyl-hexylammonium
   C₄₈H₉₂NO₈P (842,23)
89) 1-Stearoyl-2-oleoyl-sn-glycero-3-phospho-N,N,N-trimethylpropylammonium
   C₄₅H₈₈NO₈P (802,16)
90) 1-Stearoyl-2-oleoyl-sn-glycero-3-phospho-N,N,N-trimethylbutylammonium
   C₄₆H₉₀NO₈P (816,19)
91) 1-Palmitoyl-2-lauroyl-sn-glycero-3-phospho-N,N,N-trimethylpropylammonium
   C₃₇H₇₄NO₈P (691,97)
92) 1-Oleoyl-2-lauroyl-sn-glycero-3-phospho-N,N,N-trimethylpropylammonium
   C₃₉H₇₆NO₈P (718,00)
93) 1-Erucoyl-2-oleoyl-sn-glycero-3-phospho-N,N,N-trimethylpropylammonium
   C₄₉H₉₄NO₈P (856,26)
94) 1-Erucoyl-2-oleoyl-sn-glycero-3-phospho-N,N,N-trimethylbutylammonium
   C₅₀H₉₆NO₈P (870,28)
95) 1-Erucoyl-2-oleoyl-sn-glycero-3-phospho-N,N,N-trimethylhexylammonium
   C₅₂H₁₀₀NO₈P (898,34)
96) 1-Nervonoyl-2-lauroyl-sn-glycero-3-phospho-N,N,N-trimethylpropylammonium
   C₄₅H₈₈NO₈P (802,16)
97) 1-Nervonoyl-2-oleoyl-sn-glycero-3-phospho-N,N,N-trimethylpropylammonium
   C₅₁H₉₈NO₈P (884,31)

### 2) Lösungsvermittler

Es hat sich gezeigt, dass bestimmte Substanzen aus den hier vorgestellten Verbindungen insbesondere diejenigen, welche ethanollöslich sind, ausgezeichnete Lösungsvermittler für in Wasser schwerlösliche Substanzen sind. So kann man beispielsweise Taxol auf einfache Weise in eine intravenös applizierbare Form bringen. Ebenso erwiesen sich z.B. Taxoter, Cyclosporin, Cholesterin und deren Derivate, Steroide, Cortison und Analoga, Erucylphosphocholin (Auflösung der gelartigen Strukturen) als gut lösbar.

Insbesondere haben sich hier Substanzen bewährt, die zwischen Phosphat und Ammonium einen Abstand von 3 C-Atomen besitzen (n = 3 in der allgemeinen Formel I), z.B. die Substanzen aus den Beispielen 14, 85, 111, 139, 144, 176. Hervorragend geeignet für diese Zwecke ist eine einfache Substanz, 1-Erucoyl-propandiol-(1,3)-phospho-N,N,N-trimethylpropylammonium (176). Diese Substanz kann einfach in hohen Ausbeuten im Tonnenmaßstab hergestellt werden.

Bevorzugt handelt es sich bei den Lösungsvermittlern um einkettige Verbindungen, d.h. dass bei A = a einer von R₁ und R₂ Wasserstoff oder ein Alkyl mit 1 bis 3 C-Atomen darstellt.

### Taxol zur intravenösen Anwendung

Man stelle eine Lösung von 0,3 g Taxol und 1,75 g Substanz Nr. 176 in 7,95 g absolutem Ethanol her. Die Lösung wird steril filtriert und bis zur Verwendung bei 4 °C aufbewahrt.

Für eine intravenöse Gase wird die Stammlösung 1:10 oder 1:100 mit physiologischer Kochsalzlösung verdünnt.

### Beispiele für einkettige Glycero-phospho-N,N-dimethyl-N-dihydroxypropylalkylammonium-Verbindungen

(A = a; n = 2-6; R₃, CH₃; x = 0, y = 1, z = 1) 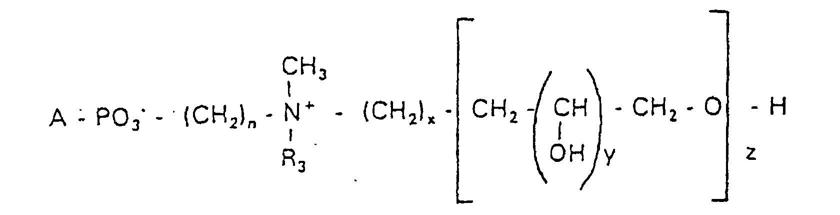
98) 1-Palmitoyl-sn-glycero-3-phospho-N,N-dimethyl-N-(diHP)-ethylammonium (n = 2)
   C₂₆H₅₄NO₉P (555,69)
99) 1-Stearoyl-sn-glycero-3-phospho-N,N-dimethyl-N-(diHP)-ethylammonium (n = 2)
   C₂₈H₅₈NO₉P (583,74)
100) 1-Arachinoyl-sn-glycero-3-phospho-N,N-dimethyl-N-(diHP)-ethylammonium (n = z)
   C₃₀H₆₂NO₉P (611,79)
101) 1-Behenyol-sn-glycero-3-phospho-N,N-dimethyl-N-(diHP)-ethylammonium (n = 2)
   C₃₂H₆₆NO₉P (639,85)
102) 1-Erucoyl-sn-glycero-3-phospho-N,N-dimethyl-N-(diHP)-ethylammonium (n = 2)
   C₃₂H₆₄NO₉P (637,83)
103) 1-Nervonoyl-sn-glycero-3-phospho-N,N-dimethyl-N-(diHP)-ethylammonium (n = 2)
   C₃₄H₆₈NO₉P (665,88)
104) 1-O-Hexadecyl-sn-glycero-3-phospho-N,N-dimethyl-N-(diHP)-ethylammonium (n = 2)
105) 1-O-Octadecyl-sn-glycero-3-phospho-N,N-dimethyl-N-(diHP)-ethylammonium (n = 2)
   C₂₈H₆₀NO₈P (569,76)
106) 1-O-Eicosanyl-sn-glycero-3-phospho-N,N-dimethyl-N-(diHP)-ethylammonium (n = 2)
   C₃₀H₆₄NO₈P (597,81)
107) 1-O-Behenyl-sn-glycero-3-phospho-N,N-dimethyl-N-(diHP)-ethylammonium (n = 2)
   C₃₂H₆₈NO₈P (625,86)
108) 1-Palmitoyl-sn-glycero-3-phospho-N,N-dimethyl-N-(diHP)-propylammonium (n = 3)
   C₂₇H₅₆NO₉P (569,71)
109) 1-Stearoyl-sn-glycero-3-phospho-N,N-dimethyl-N-(diHP)-propylammonium (n = 3)
   C₂₉H₆₀NO₉P (597,77)
110) 1-Behenoyl-sn-glycero-3-phospho-N,N-dimethyl-N-(diHP)-propylammonium (n = 3)
   C₃₃H₆₈NO₉P (653,87)
111) 1-Erucoyl-sn-glycero-3-phospho-N,N-dimethyl-N-(diHP)-propylammonium (n = 3)
   C₃₃H₆₆NO₉P (651,86)
112) 1-Nervonoyl-sn-glycero-3-phospho-N,N-dimethyl-N-(diHP)-propylammonium (n = 3)
   C₃₅H₇₀NO₉P (679,91)
113) 1-Stearoyl-sn-glycero-3-phospho-N,N-dimethyl-N-(diHP)-butylammonium (n = 4)
   C₃₀H₆₂NO₉P (611,79)
114) 1-Erucoyl-sn-glycero-3-phospho-N,N-dimethyl-N-(diHP)-butylammonium (n = 4)
   C₃₄H₆₈NO₉P (665,88)
115) 1-Stearoyl-sn-glycero-3-phospho-N,N-dimethyl-N-(diHP)-hexylammonium (n = 6)
   C₃₂H₆₆NO₉P (639,85)
116) 1-Erucoyl-sn-glycero-3-phospho-N,N-dimethyl-N-(diHP)-hexylammonium (n = 6)
   C₃₆H₇₂NO₉P (693,94)

### Beispiele für einkettige Glycero-phospho-N,N-dimethyl-N-(2-hydroxypropyl-3,1-O,O-dihydroxypropyl)-alkylammonium-Verbindungen

(A = O; n = 2-6; R₃, CH₃; x = O; y = 1; z = 2) 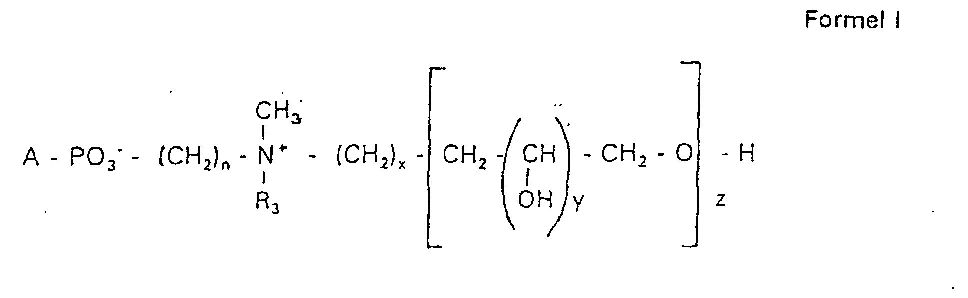
117) 1-Stearoyl-sn-glycero-3-phospho-N,N-dimethyl-N-(HP₁-diHP₂)-ethylammonium (n = 2)
   C₃₁H₆₄NO₁₁P (657,82)
118) 1-Arachinoyl-sn-glycero-3-phospho-N,N-dimethyl-N-(HP₁-diHP₂)-ethylammonium (n = 2)
   C₃₃H₆₈NO₁₁P (685,87)
119) 1-Erucoyl-sn-glycero-3-phospho-N,N-dimethyl-N-(HP₁-diHP₂)-ethylammonium (n = 2)
   C₃₅H₇₀NO₁₁P (711,91)
120) 1-Nervonoyl-sn-glycero-3-phospho-N,N-dimethyl-N-(HP₁-diHP₂)-ethylammonium (n = 2)
   C₃₇H₇₄NO₁₁P (739,96)
121) 1-O-Octadecyl-sn-glycero-3-phospho-N,N-dimethyl-N-(HP₁-diHP₂)-ethylammonium (n = 2)
   C₃₁H₆₆NO₁₀P (643,83)
122) 1-O-Behenyl-sn-glycero-3-phospho-N,N-dimethyl-N-(HP₁-diHP₂)-ethylammonium (n = 2)
   C₃₅H₇₄NO_{P} (699,94)
123) 1-Stearoyl-sn-glycero-3-phospho-N,N-dimethyl-N-(HP₁-diHP₂)-propylammonium (n = 3)
   C₃₂H₆₆NO₁₁P (671,84)
124) 1-Erucoyl-sn-glycero-3-phospho-N,N-dimethyl-N-(HP₁-diHP₂)-propylammonium (n = 3)
   C₃₆H₇₂NO₁₁P (725,94)
125) 1-Erucoyl-sn-glycero-3-phospho-N,N-dimethyl-N-(HP₁-diHP₂)-butylammonium (n = 4)
   C₃₇H₇₄NO₁₁P (739,98)
126) 1-Erucoyl-sn-glycero-3-phospho-N,N-dimethyl-N-(HP₁-diHP₂)-hexylammonium (n = 6)
   C₃₉H₇₈NO₁₁P (768,04)
127) 1-Nervonoyl-sn-glycero-3-phospho-N,N-dimethyl-N-(HP₁-diHP₂)-propylammonium (n = 3)
   C₃₈H₇₆NO₁₁P (754,01)
128) 1-Nervonoyl-sn-glycero-3-phospho-N,N-dimethyl-N-(HP₁-diHP₂)-butylammonium (n = 4)
   C₃₉H₇₈NO₁₁P (768,04)

### Beispiele für einkettige Glycero-phospho-N,N-dimethyl-N-(2-hydroxypropyl-3,10-O,O-2-hydroxypropyl-3,1-O,O-dihydroxypropyl)-alkylammonium-Verbindungen

(A = a; n = 2-6; R₃, CH₃; x = 0; y = 1; z = 3) 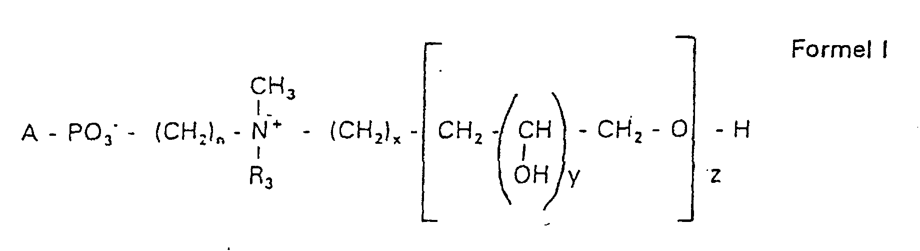
129) 1-Stearoyl-sn-glycero-3-phospho-N,N-dimethyl-N-(HP₁-HP₂-diHP₃)-ethylammonium (n = 2)
   C₃₄H₇₀NO₁₃P (731,90)
130) 1-Erucoyl-sn-glycero-3-phospho-N,N-dimethyl-N-(HP₁-HP₂-diHP₃)-ethylammonium (n = 2)
   C₂₈H₇₆NO₁₃P (785,99)
131) 1-Nervonoyl-sn-glycero-3-phospho-N,N-dimethyl-N-(HP₁-HP₂-diHP₃)-ethylammonium (n = 2)
   C₄₀H₈₀NO₁₃P (814,04)
132) 1-Erucoyl-sn-glycero-3-phospho-N,N-dimethyl-N-(HP₁-HP₂-diHP₃)-propylammonium (n = 3)
   C₃₉H₇₈NO₁₃P (800,01)
133) 1-Nervonoyl-sn-glycero-3-phospho-N,N-dimethyl-N-(HP₁-HP₂-diHP₃)-propylammonium (n = 3)
   C₄₁H₈₂NO₁₃P (828,07)
134) 1-Erucoyl-sn-glycero-3-phospho-N,N-dimethyl-N-(HP₁-HP₂-diHP₃)-butylammonium (n = 4)
   C₄₀H₈₀NO₁₃P (814,04)
135) 1-Erucoyl-sn-glycero-3-phospho-N,N-dimethyl-N-(HP₁-HP₂-diHP₃)-hexylammonium (n = 6)
   C₄₂H₈₄NO₁₃P (842,09)

### Beispiele für einkettige Glycero-phospho-Verbindungen, die nicht am Stickstoff hydroxyliert sind

(A = a; n = 3; R₃, CH₃; x = 1; z = 0) 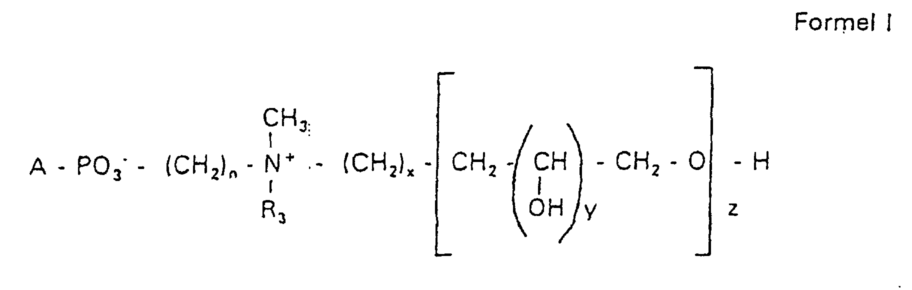
1 36) 1 -Palmitoyl-sn-glycero-3-phospho-N,N,N-trimethyl-propylammonium (n = 3)
   C₂₅H₅₂NO₇P (509,66)
137) 1-Stearoyl-sn-glycero-3-phospho-N,N,N-trimethyl-propylammonium (n = 3)
   C₂₇H₅₆NO₇P (537,71)
138) 1-Behenoyl-sn-glycero-3-phospho-N,N,N-trimethyl-propylammonium (n = 3)
   C₃₁H₆₄NO₇P (593,82)
139) 1-Erucoyl-sn-glycero-3-phospho-N,N,N-trimethyl-propylammonium (n = 3)
   C₃₁H₆₂NO₇P (591,81)
140) 1-Nervonoyl-sn-glycero-3-phospho-N,N,N-trimethyl-propylammonium (n = 3)
   C₃₃H₆₆NO₇P (619,86)

### Beispiele für ω,ω'-Alkandiol-phospho-N,N-dimethyl-N-dihydroxypropylalkylammonium-Verbindungen

(A = b; n = 2-4; R₃, CH₃; x = 0; y = 1; z = 1) 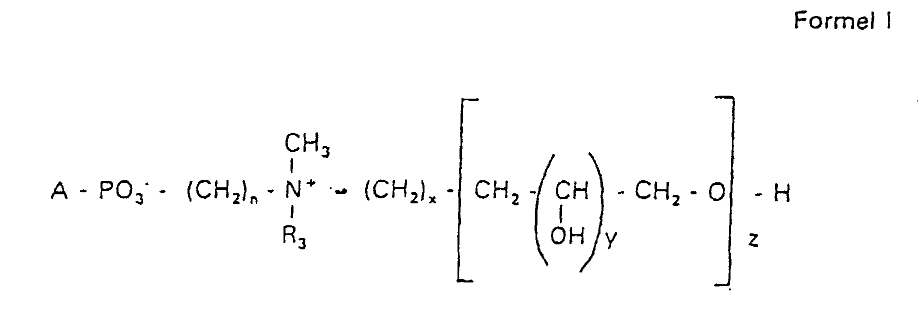
141) 1-Stearoyl-ethylenglykol-phospho-N,N-dimethyl-N-dihydroxypropylethylammonium (n = 2)
   C₂₇H₅₆NO₈P (553,71)
142) 1-Behenoyl-propandiol-(1,3)-phospho-N,N-dimethyl-N-dihydroxypropyl-ethylammonium (n = 2)
   C₃₂H₆₆NO₈P (623,85)
143) 1-Stearoyl-propandiol-(1,3)-phospho-N,N-dimethyl-N-dihydroxypropylethylammonium (n = 2)
   C₂₈H₅₈NO₈P (567,74)
144) 1-Erucoyl-propandiol-(1,3)-phospho-N,N-dimethyl-N-dihydroxypropylethylammonium (n = 2)
   C₃₂H₆₄NO₈P (621,83)
145) 1-Erucoyl-propandiol-(1,3)-phospho-N,N-dimethyl-N-dihydroxypropylpropylammonium (n = 3)
   C₃₃H₆₆NO₈P (635,86)
146) 1-Erucoyl-propandiol-(1,3)-phospho-N,N-dimethyl-N-dihydroxypropylbutylammonium (n = 4)
   C₃₄H₆₈NO₈P (649,88)

### Beispiele für Alkandiol-(1,2)-phospho-N,N-dimethyl-N-dihydroxypropylalkylammonium-Verbindungen

(A = b; n = 2-4; R₃, CH₃; x = 0; y = 1; z = 1) 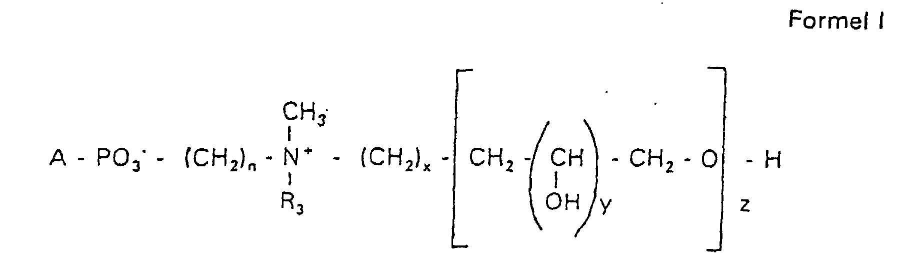
147) 2-Erucoyl-propandiol-(1,2)-phospho-N,N-dimethyl-N-dihydroxypropylethylammonium
   C₃₂H₆₄NO₈P (621,33)
148) 1 -Erucoyl-propandiol-(1,2)-phospho-N,N-dimethyl-N-dihydroxypropylethylammonium
   C₃₂H₆₄NO₈P (621,33)
149) 2-Erucoyl-propandiol-(1,2)-phospho-N,N-dimethyl-N-dihydroxypropylpropylammonium
   C₃₃H₆₆NO₈P (635,86)
150) 1 -Erucoyl-propandiol-(1,2)-phospho-N,N-dimethyl-N-dihydroxypropylbutylammonium
   C₃₄H₆₈NO₈P (649,88)

### Beispiele für ω,ω'-Alkandiol-phospho-N,N-dimethyl-N-(2-hydroxypropyl)-3,1-O,O-dihydroxypropyl)-alkylammonium-Verbindungen

(A = b; n = 2 - 4; R₃, CH₃; x = 0; y = 1; z = 2) 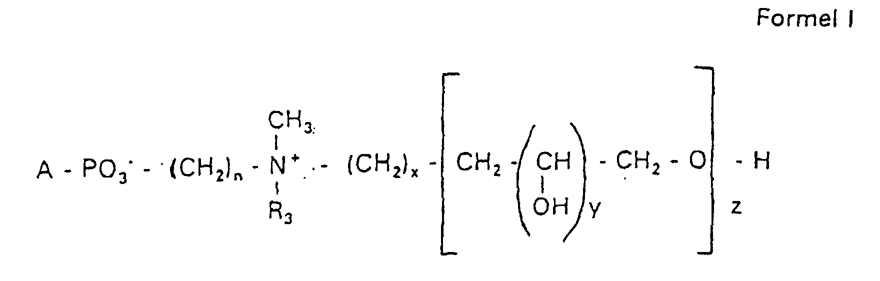
151) 1-Stearoyl-ethylenglykol-phospho-N,N-dimethyl-N-(2-hydroxypropyl-3,1-O,O-dihydroxypropyl)-ethylammonium
   C₃₀H₆₂NO₁₀P (627,79)
152) 1-Behenoyl-propandiol-(1,3)-phospho-N,N-dimethyl-N-(2-hydroxypropyl-3,1-O,O-dihydroxypropyl)-ethylammonium
   C₃₅H₇₂NO₁₀P (697,93)
153) 1-Stearoyl-propandiol-(1,3)-phospho-N,N-dimethyl-N-(2-hydroxypropyl)-3,1-O,O-dihydroxypropyl-ethylammonium
   C₃₁H₆₄NO₁₀P (641,82)
154) 1-Erucoyl-propandiol-(1,3)-phospho-N,N-dimethyl-N-(2-hydroxypropyl-3,1-O,O-dihydroxypropyl)-ethylammonium
   C₃₅H₇₀NO₁₀P (695,91)
155) 1-Erucoyl-propandiol-(1,3)-phospho-N,N-dimethyl-N-(2-hydroxypropyl-3,1-O,O-dihydroxypropyl)-propylammonium
   C₃₆H₇₂NO₁₀P (709,94)
156) 1-Erucoyl-propandiol-(1,3)-phospho-N,N-dimethyl-N-(2-hydroxypropyl-3,1-O,O-dihydroxypropyl)-butylammonium
   C₃₇H₇₄NO₁₀P (723,96)
157) 1-Erucoyl-butandiol-(1,4)-phospho-N,N-dimethyl-N-(2-hydroxypropyl-3,1-O,O-dihydroxypropyl)-propylammonium
   C₃₇H₇₄NO₁₀P (723,96)
158) 1-Erucoyl-hexandiol-(1,6)-phospho-N,N-dimethyl-N-(2-hydroxypropyl-3,1-O,O-dihydroxypropyl)-propylammonium
   C₃₉H₇₈NO₁₀P (752,02)
159) 1-Erucoyl-octandiol-(1,8)-phospho-N,N-dimethyl-N-(2-hydroxypropyl)-3,1-0,0-dihydroxypropyl)-propylammonium
   C₄₁H₈₂NO₁₀P (780,07)

### Beispiele für Alkandiol-(1,2)-phospho-N,N-dimethyl-N-(2-hydroxypropyl-3,1-0,0-dihydroxypropyl)-alkylammonium-Verbindungen

(A = b; n = 2 - 4; R₃, CH₃; x = 0; y = 1; z = 2) 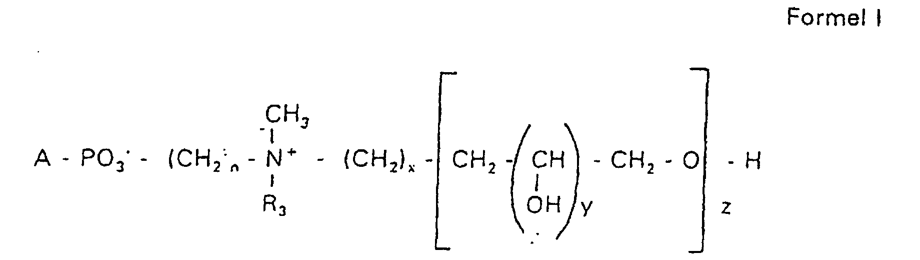
160) 2-Erucoyl-propandiol-(1,2)-phospho-N,N-dimethyl-N-(2-hydroxypropyl-3,1-0,0-dihydroxypropyl)-ethylammonium
   C₃₅H₇₀NO₁₀P (695,91)
161) 1-Erucoyl-propandiol-(1,2)-phospho-N,N-dimethyl-N-(2-hydroxypropyl-3,1-O,O-dihydroxypropyl)-ethylammonium
   (C₃₅H₇₀NO₁₀P (695,91)
1 62) 2-Erucoyl-propandiol-(1,2)-phospho-N,N-dimethyl-N-(2-hydroxypropyl-3,1-O,O-dihydroxypropyl)-propylammonium
   C₃₆H₇₂NO₁₀P (709,94)
1 63) 1-Erucoyl-propandiol-(1,2)-phospho-N,N-dimethyl-N-(2-hydroxypropyl-3,1-O,O-dihydroxypropyl)-butylammonium
   C₃₇H₇₄NO₁₀P (723,96)
164) 1-Erucoyl-butandiol-(1,2)-phospho-N,N-dimethyl-N-(2-hydroxypropyl-3,1-O,O-dihydroxypropyl)-propylammonium
   C₃₇H₇₄NO₁₀P (723,96)
165) 1 -Erucoyl-hexandiol-(1,2)-phospho-N,N-dimethyl-N-(2-hydroxypropyl-3,1-O,O-dihydroxypropyl)-propylammonium
   C₃₉H₇₈NO₁₀P (752,02)
166) 1-Erucoyl-octandiol-(1,2)-phospho-N,N-dimethyl-N-(2-hydroxypropyl-3,1-O,O-dihydroxypropyl)-propylammonium
   C₄₁H₈₂NO₁₀P (780,07)

### Beispiele für ω,ω'-Alkandiol-N,N-dimethyl-N-(2-hydroxypropyl-3,1-O,O-2-hydroxypropyl-3,1-O,O-dihydroxypropyl)-alkylammonium-Verbindungen

(Abkürzung: HP = 2-Hydroxypropyl
diHP = Dihydroxypropyl)
(A = b; n = 2 - 6; R, CH₃; x = 0; y = 1; z = 3) 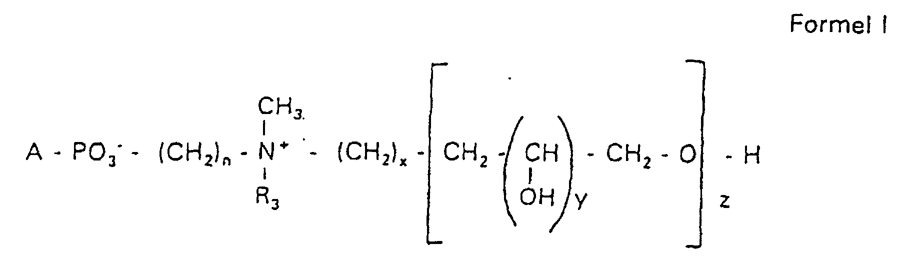
167) 1-Oleoyl-ethylenglykol-phospho-N,N-dimethyl-N-(HP₁-HP₂-diHP₃)-ethylammonium
   C₃₄H₆₈NO₁₂P (713,88)
168) 1-Erucoyl-propandiol-(1,3)-phospho-N,N-dimethyl-N-(HP₁-HP₂-diHP₃)-ethylammonium
   C₃₈H₇₆NO₁₂P (769,99)
169) 1-Oleoyl-propandiol-(1,3)-phospho-N,N-dimethyl-N-(HP₁-HP₂-diHP₃)-propylammonium
   C₃₅H₇₀NO₁₂P (727,91)
170) 1-Erucoyl-propandiol-(1,3)-phospho-N,N-dimethyl-N-(HP₁-HP₂-diHP₃)-propylammonium
   C₃₉H₇₈NO₁₂P (784,01)
171) 1-Erucoyl-propandiol-(1,3)-phospho-N,N-dimethyl-N-(HP₁-HP₂-diHP₃)-butylammonium
   C₄₀H₈₀NO₁₂P (798,04)
172) 1-Erucoyl-propandiol-(1,3)-phospho-N,N-dimethyl-N-(HP₁-HP₂ diHP₃)-hexylammonium
   C₄₂H₈₄NO₁₂P (826,10)

### Beispiele für Alkandiol-phospho-Verbindungen, die nicht am Stickstoff hydroxyliert sind

(A = b; n = 2 - 6; R, CH₃; x = 1; z = 0) 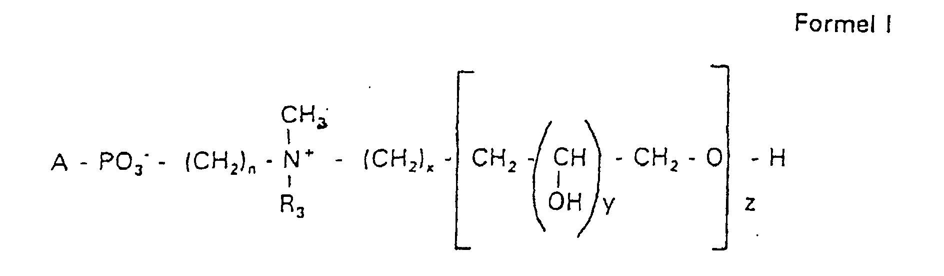
173) 1-Erucoyl-ethylenglykol-phospho-N,N,N-trimethyl-propylammonium
   C₃₀H₆₀NO₆P (561,78)
174) 1-Arachinoyl-propandiol-(1,3)-phospho-N,N,N-trimethylpropylammonium
   C₂₉H₆₉NO₆P (549,77)
175) 1-Stearoyl-propandiol-(1,3)-phospho-N,N,N-trimethylpropylammonium
   C₂₇H₅₆NO₆P (521,71)
176) 1-Erucoyl-propandiol-(1,3)-phospho-N,N,N-trimethyl-propylammonium
   C₃₁H₆₂NO₆P (575,81)
177) 1-Erucoyl-propandiol-(1,3)-phospho-N,N,N-trimethyl-butylammonium
   C₃₂H₆₄NO₆P (589,83)
178) 1 -Erucoyl-propandiol-(1,3)-phospho-N,N,N-trimethyl-pentylammonium
   C₃₃H₆₆NO₆ (603,86)
179) 1-Erucoyl-propandiol-(1,2)-phospho-N,N,N-trimethyl-propylammonium
   C₃₁H₆₂NO₆P (575,81)
180) 2-Erucoyl-propandiol-(1,2)-phospho-N,N,N-trimethyl-propylammonium
   C₃₁H₆₂NO₆P (575,81)
181) 1-Erucoyl-propandiol-(1,2)-phospho-N,N,N-trimethyl-butylammonium
   C₃₂H₆₄NO₆P (589,83)
182) 1-Erucoyl-propandiol-(1,2)-phospho-N,N,N-trimethyl-hexylammonium
   C₃₄H₆₈NO₈P (617,92)

### 3) Wirkstoffe

In früheren Untersuchungen wurde festgestellt, dass Alkylphosphocholine nur dann antitumorale Wirksamkeit besitzen, wenn der Phosphat-Ammoniumabstand zwei C-Atome beträgt, also Phosphocholin (n = 2 in der allgemeinen Formel I) entspricht. Verbindungen mit dem Abstand n > 2 waren unwirksam. Bei diesen früheren Untersuchungen wurden die Wirkstoffe oral appliziert.

Überraschenderweise haben wir nun festgestellt, dass Erucylphospho-Verbindungen mit Phosphat-Ammoniumabständen > 2 exzellente, den Alkylphosphocholinen sogar überlegene Antitumorwirksamkeit besitzen, wenn diese Substanzen intravenös angewendet werden, wie folgender Vergleich zeigt:

### Erucylphosphocholin (n = 2 in allgemeiner Formel I)

Die Substanz bildet in Wasser gelartige Strukturen und ist deshalb in höheren Konzentrationen intravenös nur schwer anwendbar. Eurcylphosphocholin besitzt im Tiermodell Methylnitrosoharnstoffinduziertes Mammakarzinom nur eine geringe Langzeitwirkung. Bereits 7 Tage nach Absetzen der Therapie wird wieder Tumorwachstum beobachtet.

### Erucyl-phospho-N,N,N-trimethyl-propylammonium (n = 3)

Die Substanz ist in Wasser leicht löslich, bildet keine Gele und kann problemlos intravenös verabreicht werden. Sie kann deshalb uach als Lösungsvermittler Verwendung finden. Insbesondere auffällig und eindrucksvoll ist aber ihre Langzeitwirkung in obigem Tiermodell. Selbst 4 Wochen nach Absetzen der Therapie ist kein neues Tumorwachstum zu beobachten.

### Beispiele für Alkyl-phospho-N,N-dimethyl-N-dihydroxypropylalkylammonium-Verbindungen

(A = c; n = 2 - 6; R₃, CH₃; x = 0; y = 1; z = 1) 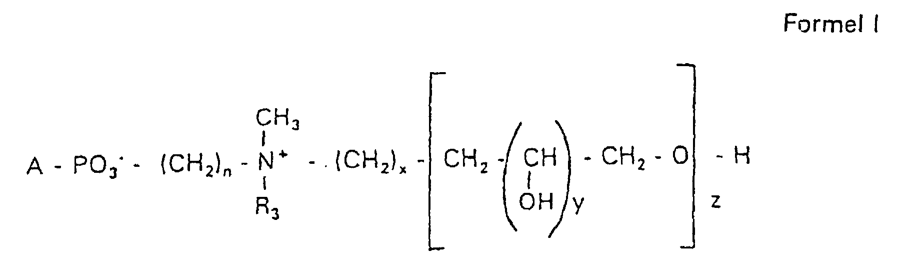
183) Hexadecyl-phospho-N,N-dimethyl-N-dihydroxypropyl-ethylammonium
   C₂₃H₅₀NO₆P (467,62)
184) Octadecyl-phospho-N,N-dimethyl-N-dihydroxypropyl-ethylammonium
   C₂₅H₅₄NO₆P (495,68)
185) Erucyl-phospho-N,N-dimethyl-N-dihydroxypropyl-ethylammonium
   C₂₉H₆₀NO₆P (549,77)
186) Erucyl-phospho-N,N-dimethyl-N-dihydroxypropyl-propylammonium
   C₃₀H₆₂NO₆P (563,80)
187) Erucyl-phospho-N,N-dimethyl-N-dihydroxypropyl-butylammonium
   C₃₁H₆₄NO₆P (577,82)
188) Erucyl-phospho-N,N-dimethyl-N-dihydroxypropyl-hexylammonium
   C₃₃H₆₈NO₆P (605,88)
189) Oleyl-phospho-N,N-dimethyl-N-dihydroxypropyl-ethylammonium
   C₂₅H₅₂NO₆P (493,66)
190) Oleyl-phospho-N,N-dimethyl-N-dihydroxypropyl-propylammonium
   C₂₆H₅₄NO₆P (507,69)
191) Oleyl-phospho-N,N-dimethyl-N-dihydroxypropyl-butylammonium
   C₂₇H₅₆NO₆P (521,21)
192) (Z-11)-Eicosenyl-phospho-N,N-dimethyl-N-dihydroxypropylethylammonium
   C₂₇H₅₆NO₆P (521,21)
193) (Z-11)-Eicosenyl-phospho-N,N-dimethyl-N-dihydroxypropylpropylammonium
   C₂₈H₅₈NO₆P (535,74)
194) (Z-1 1 )-Eicoseny|-phospho-N,N-dimethyl-N-dihydroxypropylbutylammonium
   C₂₉H₆₀NO₆P (549,77)
195) (Z-11)-Eicosenyl-phospho-N,N-dimethyl-N-dihydroxypropylpentylammonium
   C₃₀H₆₂NO₆P (563,80)
196) (Z-11)-Eicosenyl-phospho-N,N-dimethyl-N-dihydroxypropylhexylammonium
   C₃₁H₆₄NO₆P (577,82)

### Beispiele für Alkyl-phospho-N,N-dimethyl-N-(2-hydroxypropyl-1,2-dihydroxypropyl)-alkylammonium

(A = c; n = 2 - 6; R₃, CH₃; x = 0; y = 1; z = 2) 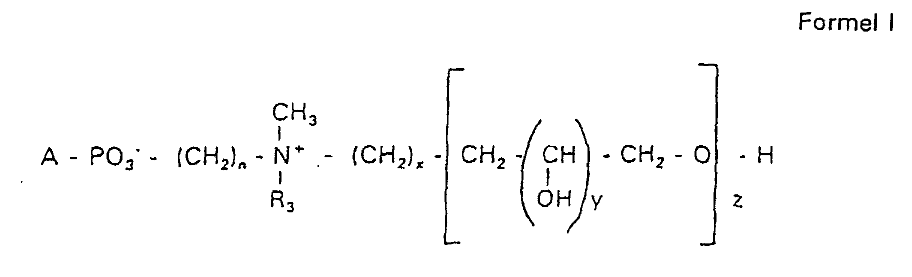
197) Hexadecyl-phospho-N,N-dimethyl-N-(HP₁-diHP₂)-ethylammonium
   C₂₆H₅₆NO₈P (541,70)
198) Octadecyl-phospho-N,N-dimethyl-N-(HP₁-diHP₂)-ethylammonium
   C₂₈H₆₀NO₈P (569,76)
199) Erucyl-phospho-N,N-dimethyl-N-(HP₁-diHP₂)-ethylammonium
   C₃₂H₆₆NO₈P (623,85)
200) Erucyl-phospho-N,N-dimethyl-N-(HP₁-diHP₂)-propylammonium
   C₃₃H₆₈NO₈P (637,87)
201) Erucyl-phospho-N,N-dimethyl-N-(HP₁-diHP₂)-butylammonium
   C₃₄H₇₀NO₈P (651,90)
202) Erucyl-phospho-N,N-dimethyl-N-(HP₁-diHP₂)-hexylammonium
   C₃₆H₇₄NO₈P (679,95)
203) Oleyl-phospho-N,N-dimethyl-N-(HP₁-diHP₂)-ethylammonium
   C₂₈H₅₈NO₈P (567,74)
204) Oleyl-phospho-N,N-dimethyl-N-(HP₁-diHP₂)-propylammonium
   C₂₉H₆₀NO₈P (581,77)
205) Oleyl-phospho-N,N-dimethyl-N-(HP₁-diHP₂)-butylammonium
   C₃₀H₆₂NO₈P (595,79)
206) (Z-11)-Eicosenyl-phospho-N,N-dimethyl-N-(HP₁-diHP₂)-ethylammonium
   C₃₀H₆₂NO₈P (595,79)
207) (Z-11)-Eicosenyl-phospho-N,N-dimethyl-N-(HP₁-diHP₂)-propylammonium
   C₃₀H₆₄NO₈P (609,82)
208) (Z-11)-Eicosenyl-phospho-N,N-dimethyl-N-(HP₁-diHP₂)-butylammonium
   C₃₂H₆₆NO₈P (623,85)

### Beispiele für Alkyl-phospho-N,N-dimethyl-N-(2-hydroxypropyl-3,1-0,0-hydroxypropyl-3,1-O,O-1,2-dihydroxypropyl)-alkylammonium-Verbindungen

(A = c; n = 2 - 4; R₃, CH₃; x = 0; y = 1; z = 3) 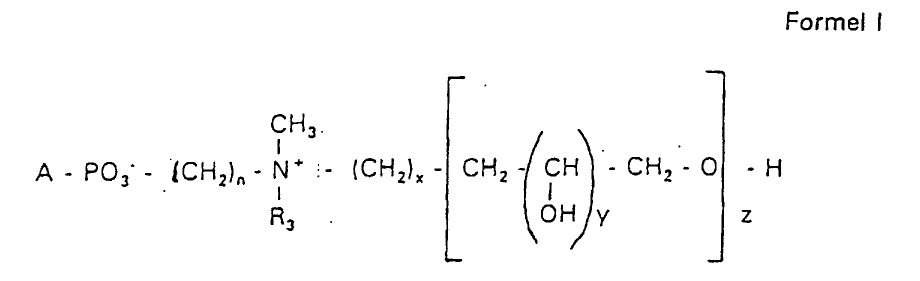
209) Hexadecyl-phospho-N,N-dimethyl-N-(HP₁-HP₂-diHP₃)-ethylammonium
   C₂₉H₆₂NO₁₀P (615,78)
210) Octadecyl-phospho-N,N-dimethyl-N-(HP₁-HP₂-diHP₃)-ethylammonium
   C₃₁H₆₅NO₁₀P (643,83)
211) Oleol-phospho-N,N-dimethyl-N-(HP₁-HP₂-diHP₃)-ethylammonium
   C₃₁H₆₄NO₁₀P (641,82)
212) (Z-11)-Eicosenyl-phospho-N,N-dimethyl-N-(HP₁-HP₂-diHP₃)-ethylammonium
   C₃₃H₆₈NO₁₀P (669,87)
213) (Z-11)-Eicosenyl-phospho-N,N-dimethyl-N-(HP₁-HP₂-diHP₃)-propylammonium
   C₃₄H₇₀NO₁₀P (683,90)
214) (Z-11)-Eicosenyl-phospho-N,N-dimethyl-N-(HP₁-HP₂-diHP₃)-butylammonium
   C₃₅H₇₂NO₁₀P (697,93)
215) Erucyl-phospho-N,N-dimethyl-N-(HP₁-HP₂-diHP₃)-ethylammonium
   C₃₅H₇₂NO₁₀P (697,93)
216) Erucyl-phospho-N,N-dimethyl-N-(HP₁-HP₂-diHP₃)-Propylammonium
   C₃₆H₇₄NO₁₀P (711,95)

### Beispiele für Alkylphospho-Verbindungen, die am Stickstoff keine Dihydroxyalkylreste tragen

(A = a; n = 3-6; R₃, CH₃; x = 1-3; z = 0) 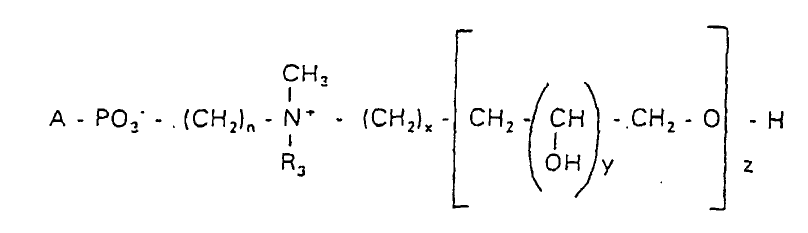
217) Eurcyl-phospho-N,N,N-trimethyl-propylammonium
   C₂₈H₅₈NO₄P (503,74)
218) Erucyl-phospho-N,N-dimethyl-N-ethyl-propylammonium
   C₂₉H₆₀NO₄P (517,77)
219) Erucyl-phospho-N,N-dimethyl-N-propyl-propylammonium
   C₃₀H₆₂NO₄P (531,80)
220) Erucyl-phospho-N,N-dimethyl-N-allyl-propylammonium
   C₃₀H₆₀NO₄P (529,78)
   Rf-Werte der Substanzen 217-240 im beschriebenen System: Rf 0,30-0,40
221) (Z)-10-Docosenyl-2-phospho-N,N,N-trimethyl-propylammonium
   C₂₈H₅₈NO₄P (503,74)
222) (Z)-10-Docosenyl-2-phospho-N,N-dimethyl-N-ethyl-propylammonium
   C₂₉H₆₀NO₄P (517,77)
223) Erucyl-phospho-N,N,N-trimethyl-butylammonium
   C₂₉H₆₀NO₄P (517,77)
224) Erucyl-phospho-N,N-dimethyl-N-ethyl-butylammonium
   C₃₀H₆₂NO₄P (531,80)
225) Erucyl-phospho-N,N-dimethyl-N-propyl-butylammonium
   C₃₁H₆₄NO₄P (545,82)
226) (Z)-10-Docosenyl-2-phospho-N,N,N-trimethyl-butylammonium
   C₂₉H₆₀NO₄P (517,77)
227) (2)-11-Eicosenyl-phospho-N,N,N-trimethyl-propylammonium
   C₂₆H₅₄NO₄P (475,69)
228) (Z)-11-Eicosenyl-phospho-N, N-dimethyl-N-ethyl-propylammonium
   C₂₇H₅₆NO₄P (489,72)
229) (Z)-11-Eicosenyl-phospho-N,N-diethyl-N-methyl-propylammonium
   C₂₈H₅₈NO₄P (503,74)
230) (Z)-11-Eicosenyl-phospho-N,N-dimethyl-N-propyl-propylammonium
   C₂₈H₅₈NO₄P (503,74)
231) (Z)-11-Eicosenyl-phospho-N,N,N-trimethyl-butylammonium
   C₂₇H₅₆NO₄P (489,72)
232) (Z)-11-Eicosenyl-phospho-N,N-dimethyl-N-ethyl-butylammonium
   C₂₈H₅₈NO₄P (503,74)
233) (Z)-11-Eicosenyl-phospho-N,N-dimethyl-N-propyl-butylammonium
   C₂₉H₆₀NO₄P (517,77)
234) (Z)-11-Eicosenyl-phospho-N,N-dimethyl-N-alkyl-butylammonium
   C₂₉H₅₈NO₄P (575,75)
235) Oleyl-phospho-N,N,N-trimethyl-propylammonium
   C₂₄H₅₀NO₄P (447,64)
236) Oleyl-phospho-N,N-dimethyl-N-ethyl-propylammonium
   C₂₅H₅₂NO₄P (461,66)
237) Oleyl-phospho-N,N-dimethyl-N-propylen-propylammonium
   C₂₆H₅₄NO₄P (475,69)
238) Oleyl-phospho-N,N,N-trimethyl-butylammonium
   C₂₅H₅₂NO₄P (461,66)
239) Oleyl-phospho-N,N-dimethyl-N-ethyl-butylammonium
   C₂₆H₅₄NO₄P (475,69)
240) Oleyl-phospho-N,N-dimethyl-N-propyl-butylammonium
   C₂₇H₅₆NO₄P (489,72)

### Wirkstoffe, die auf alkylierten (Ether)-Lysolecithinen aufgebaut und am Stickstoff hydroxyliert sind

(A = a; n = 2 - 4; R₃, CH₃; x = 0; y = 1; z = 1, 2) 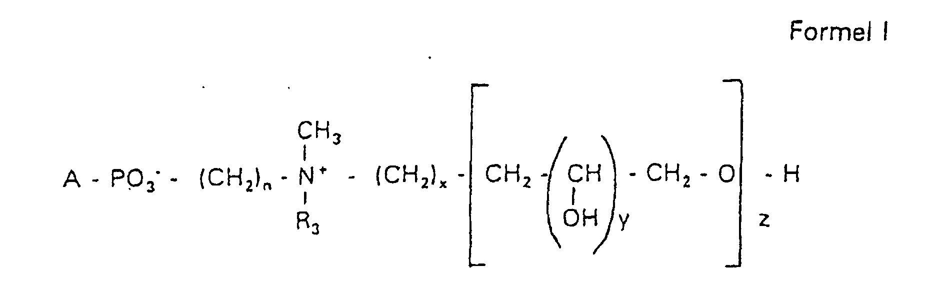
241) 1-O-Octadecyl-2-0-methyl-sn-glycero-3-phospho-N,N-dimethyl-N-dihydroxypropyl-ethylammonium (n = 2)
   C₂₉H₆₂NO₈P (583,78)
242) 3-0-Octadecyl-2-0-methyl-sn-glycero-1-phospho-N,N-dimethyl-N-dihydroxypropyl-ethylammonium (n = 2)
   C₂₉H₆₂NO₈P (583,78)
243) 1-O-Octadecyl-2-0-tert.butyl-sn-glycero-3-phospho-N,N-dimethyl-N-dihydroxypropyl-ethylammonium (n = 2)
   C₃₂H₆₈NO₈P (625,86)
244) 3-0-Octadecyl-2-0-tert.butyl-sn-glycero-3-phospho-N,N-dimethyl-N-dihydroxypropyl-ethylammonium (n = 2)
   C₃₂H₆₈NO₈P (625,86)
245) 1-0-Octadecyl-2-0-methyl-sn-glycero-3-phospho-N,N-dimethyl-N-dihydroxypropyl-propylammonium (n = 3)
   C₃₀H₆₄NO₈P (597,81)
246) 1-0-Octadecyl-2-0-methyl-sn-glycero-3-phospho-N,N-dimethyl-N-dihydroxypropyl-butylammonium (n = 4)
   C₃₁H₆₆NO₈P (611,84)
247) 1-0-Erucyl-2-0-methyl-sn-glycero-3-phospho-N,N-dimethyl-N-dihydroxypropyl-ethylammonium (n = 2)
   C₃₃H₆₈NO₈P (637,87)
248) 1-O-Erucyl-2-O-methyl-sn-glycero-3-phospho-N,N-dimethyl-N-dihydroxypropyl-propylammonium (n = 3)
   C₃₄H₇₀NO₈P (651,90)
249) 1-0-Octadecyl-2-0-methyl-sn-glycero-3-phospho-N,N-dimethyl-N-(HP₁diHP₂)-ethylammonium in = 2)
   C₃₂H₆₈NO₁₀P (657,86)
250) 1-0-Octadecyl-2-0-tert.butyl-sn-glycero-3-phospho-N,N-dimethyl-N-(HP₁-diHP₂)-ethylammonium (n = 2)
   C₃₅H₇₄NO₁₀P (699,94)
251) 1-0-Octadecyl-2-0-methyl-sn-glycero-3-phospho-N,N-dimethyl-N-(HP₁diHP₂)-propylammonium (n = 3)
   C₃₃H₇₀NO₁₀P (671,89)
252) 1-O-Octadecyl-2-0-tert.butyl-sn-glycero-3-phospho-N,N-dimethyl-N-(HP₁-diHP₂)-propylammonium (n = 3)
   C₃₆H₇₆NO₁₀P (713,97)
253) 1-0-Octadecyl-2-0-tert.butyl-sn-glycero-3-phospho-N,N-dimethyl-N-(HP₁-diHP₂)-butylammonium (n = 4)
   C₃₇H₇₈NO₁₀P (727,99)
254) 1-0-Erucyl-2-0-methyl-sn-glycero-3-phospho-N,N-dimethyl-N-(HP₁diHP₂)-butylammonium (n = 4)
   C₃₈H₇₈NO₁₀P (739,01)

### Wirkstoffe, die auf alkylierten (Ether)-Lysolecithinen aufgebaut sind und am Stickstoff nicht hydroxyliert sind

(A = a; n = 3, 4; R₃, CH₃; x = 1; z = 0) 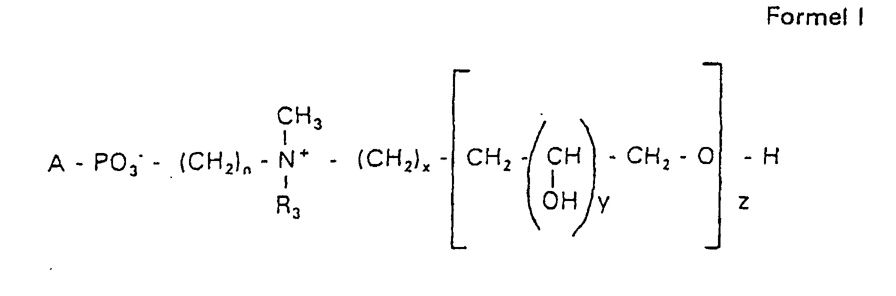
255) 1-0-Erucyl-2-0-methyl-sn-glycero-3-phospho-N,N,N-trimethylpropylammonium (n = 3)
   C₃₂H₆₆NO₆P (591,85)
256) 1-0-Erucyl-3-0-methyl-sn-glycero-2-phopsho-N,N,N-trimethylpropylammonium (n = 3)
   C₃₂H₆₆NO₆P (591,85)
257) 1-0-(Z)-11-Eicosenyl-2-0-methyl-sn-glycero-3-phospho-N,N,N-trimethyl-propylammonium (n = 3)
   C₃₀H₆₂NO₆P (563,80)
258) 1-O-(Z)-11-Eicosenyl-2-0-tert.butyl-sn-glycero-3-phospho-N,N,N-trimethyl-propylammonium (n = 3)
   C₃₃H₆₈NO₆P (605,88)
259) 1-0-Oleyl-2-0-tert.butyl-sn-glycero-3-phospho-N,N,N-trimethylpropylammonium (n = 3)
   C₃₁H₆₄NO₆P (577,82)
260) 1-0-(Z)-11-Eicosenyl-2-0-tert.butyl-sn-glycero-3-phospho-N,N,N-butylammonium (n = 4)
   C₃₄H₇₀NO₆P (619,90)
261) 1-O-Oleyl-2-0-tert.butyl-sn-glycero-3-phospho-N,N,N-trimethylbutylammonium (n = 4)
   C₃₂H₆₆NO₆P (591,85)

### Wirkstoffe, die auf Alkandiolphospho-Verbindungen aufgebaut und am Stickstoff hydroxyliert sind

(A = b; n = 2, 3; R₃, CH₃; x = 0; y = 1; z = 1) 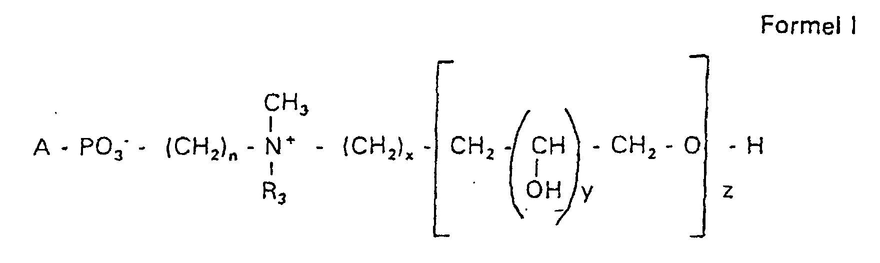
262) 1-0-Erucyl-ethylenglykol-phospho-N,N-dimethyl-N-dihydroxypropylethylammonium
   C₃₁H₆₄NO₇P (593,82)
263) 1-O-Erucyl-propandiol-(1,3)-phospho-N,N-dimethyl-N-dihydroxypropylethylammonium
   C₃₂H₆₆NO₇P (607,85)
264) 1-O-Erucyl-propandiol-(1,2)-phospho-N,N-dimethyl-N-dihydroxypropylethylammonium
   C₃₂H₆₆NO₇P (607,85)
265) 2-0-Erucyl-propandiol-(1,2)-phospho-N,N-dimethyl-N-dihydroxypropylethylammonium
   C₃₂H₆₆NO₇P (607,85)
266) 1-O-(Z)-11-Eicosenyl-propandiol-(1,2)-phospho-N,N-dimethyl-N-dihydroxypropyl-ethylammonium
   C₃₀H₆₂NO₇P (579,49)
267) 2-0-(Z)-11-Eicosenyl-propandiol-(1,2)-phospho-N,N-dimethyl-N-dihydroxypropyl-ethylammonium
   C₃₀H₆₂NO₇P (579,49)
268) 1-0-Oleyl-propandiol-(1,2)-phospho-N,N-dimethyl-N-dihydroxypropylethylammonium
   C₂₈H₅₈NO₇P (551,74)
269) 2-0-Oleyl-propandiol-(1,2)-phospho-N,N-dimethyl-N-dihydroxypropylethylammonium
   C₂₈H₅₈NO₇P (551,74)
270) 2-0-Octadecyl-propandiol-(1,2)-phospho-N,N-dimethyl-N-dihydroxypropyl-ethylammonium
   C₂₈H₆₀NO₇P (553,76)
271) 1-0-Octadecyl-propandiol-(1,2)-phospho-N,N-dimethyl-N-dihydroxypropyl-propylammonium
   C₂₉H₆₂NO₇P (626,24)
272) 2-0-Octadecyl-propandiol-(1,2)-phospho-N,N-dimethyl-N-dihydroxypropyl-propylammonium
   C₂₉H₆₂NO₇P (626,24)

### Wirkstoffe, die auf Alkandiolphospho-Verbindungen aufgebaut und am Stickstoff nicht hydroxyliert sind

(A = b; n = 3; R₃, CH₃; x = 1; z = 0) 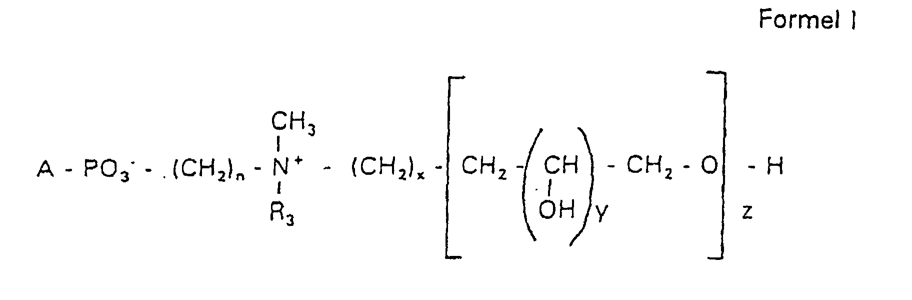
273) 1-0-Erucyl-propandiol-(1,2)-phospho-N,N,N-trimethylpropylammonium
   C₃₁H₆₄NO₅P (562,82)
274) 1-0-Erucyl-propandiol-(1,2)-phospho-N,N,N-trimethylpropylammonium.
   C₃₁H₆₄NO₅P (562,82)
275) 1-0-(Z)-1-Eicosenyl-propandiol-(1,3)-phospho-N,N,N-trimethylpropylammonium
   C₂₉H₆₀NO₅P (533,77)
276) 1-0-Oleyl-propandiol-(1,2)-phospho-N,N,N-trimethyl-propylammonium
   C₂₇H₅₆NO₅P (505,72)
277) 2-0-Oleyl-propandiol-(1,2)-phospho-N,N,N-trimethyl-propylammonium
   C₂₇H₅₆NO₅P (505,72)
278) 1-0-Octadecyl-propandiol-(1,2)-phospho-N,N,N-propylammonium
   C₂₇H₅₈NO₅P (507,73)
279) 2-0-Octadecyl-propandiol-(1,2)-phospho-N,N,N-trimethylpropylammonium
   C₂₇H₅₈NO₅P (507,73)

## Patentansprüche

1. Verbindung der allgemeinen Formel (I) 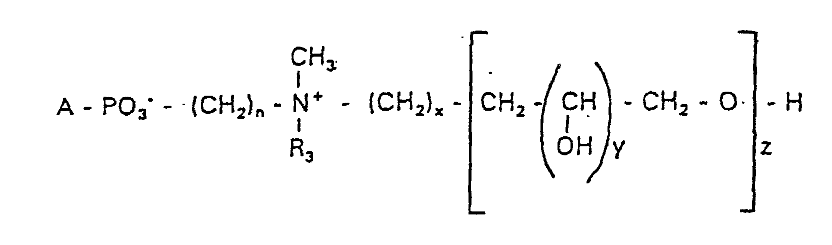 worin A 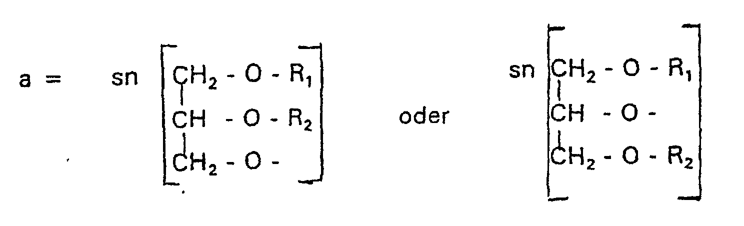 oder 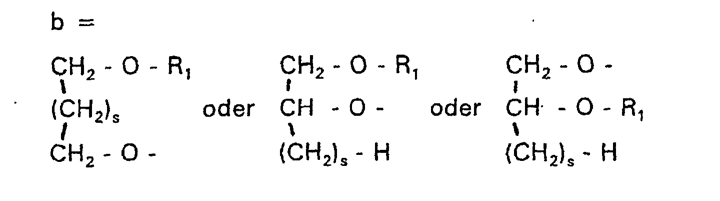 ist, wobei R₁ und R₂ unabhängig voneinander Wasserstoff, einen gesättigten oder ungesättigten Acyl- oder Alkylrest bedeuten, der gegebenenfalls verzweigt oder/und substituiert sein kann, und die Summe der Kohlenstoffatome in Acyl und Alkyl 16 bis 44 C-Atome beträgt,
s eine ganze Zahl von 0 bis 8 darstellt,
c = ein Rest eines primären oder sekundären Alkohols der Formel RO- ist, wobei R einen gesättigten oder ungesättigten Alkylrest vorwiegend mit cis-Doppelbindung, von 12 bis 30 Kohlenstoffatomen bedeutet,
n eine ganze Zahl von 2 bis 8 darstellt,
R₃ a = 1,2-Dihydroxypropyl sein kann oder
b = Alkyl mit 1 bis 3 C-Atomen sein kann, wenn z > 0 ist oder
c = Alkyl mit 1 bis 3 C-Atomen sein kann, wenn n ≠ 2 und z = 0 ist,
x = eine ganze Zahl von 0 bis 8 darstellt,
y = 1 für z = 1 bis 5 ist oder
= 1 bis 4 ist für z = 1
z = eine ganze Zahl von 0 bis 5 darstellt.

2. Verbindung nach Anspruch 1 mit
x = 0
y = 1
z = 1.

3. Verbindung nach Anspruch 1 mit
x = 0
y = 1
z = 2.

4. Verbindung nach Anspruch 1 mit
x = 0
y = 1
z = 3.

5. Verbindung nach Anspruch 1 mit
x = 0
y = 1
z = 4.

6. Verbindung nach Anspruch 1 mit
x = 1 - 3
z = 0.

7. Verbindung nach Anspruch 6 mit
x = 1
z = 0.

8. Verbindung nach einem der vorhergehenden Ansprüche mit
n = 2 - 6.

9. Verbindung nach Anspruch 8 mit
n = 2 - 4.

10. Verbindung nach einem der vorhergehenden Ansprüche, worin R₃ einen CH₃-Rest darstellt.

11. Verbindung nach einem der vorhergehenden Ansprüche,
**dadurch gekennzeichnet,**
**daß** A eine Gruppe der Formel a darstellt.

12. Verbindung nach einem der Ansprüche 1 bis 11,
**dadurch gekennzeichnet,**
**daß** A eine Gruppe der Formel a darstellt und einer von R₁ und R₂ H oder eine Alkylkette mit 1 - 3 C-Atomen ist.

13. Verbindung nach einem der vorhergehenden Ansprüche,
**dadurch gekennzeichnet,**
**daß** A eine Gruppe der Formel a darstellt und
n = 3 - 6.

14. Verbindung nach Anspruch 13,
**dadurch gekennzeichnet,**
**daß**
n=3.

15. Verbindung nach Anspruch 13,
**dadurch gekennzeichnet,**
**daß**
n = 4.

16. Verbindung nach einem der Ansprüche 1 bis 10,
**dadurch gekennzeichnet,**
**daß** A eine Gruppe der Formel b darstellt.

17. Verbindung nach Anspruch 11,
**dadurch gekennzeichnet,**
**daß**
n = 2.

18. Verbindung nach einem der Ansprüche 1 bis 10,
**dadurch gekennzeichnet,**
**daß**
n = 3.

19. Verbindung nach einem der Ansprüche 1 bis 10,
**dadurch gekennzeichnet,**
**daß**
n = 4.

20. Verbindung nach einem der Ansprüche 1 bis 10,
**dadurch gekennzeichnet,**
**daß** A eine Gruppe der Formel c darstellt.

21. Verbindung nach Anspruch 20,
**dadurch gekennzeichnet,**
**daß**
n = 3.

22. Liposome, die Phospholipide oder/und Alkylphospholipide, gegebenenfalls Cholesterin und 1 bis 50 Mol-% einer Verbindung der allgemeinen Formel (I) von Anspruch 1 oder deren Salze enthalten, wobei das Cholesterin, die Phospholipide, die Alkylphospholipide und die Verbindung der Formel (I) zusammen 100 Mol-% ergeben.

23. Verwendung einer Verbindung der allgemeinen Formel (I) von Anspruch 1 als Gentransportvehikel.

24. Verwendung einer Verbindung der allgemeinen Formel (I) von Anspruch 1 als Lösungsmittel für wasserunlösliche Wirkstoffe.

25. Verwendung nach Anspruch 24,
**dadurch gekennzeichnet,**
**dass** R¹ einen Erucasäurerest darstellt.

26. Pharmazeutische Zusammensetzung,
**dadurch gekennzeichnet,**
**daß** sie Liposomen nach Anspruch 22, in denen ein oder mehrere pharmazeutische Wirkstoffe eingeschlossen sind, gegebenenfalls zusammen mit pharmazeutisch üblichen Verdünnungs-, Hilfs-, Trägerund Füllstoffen enthält.

## Claims

1. Compound of the general formula (I) 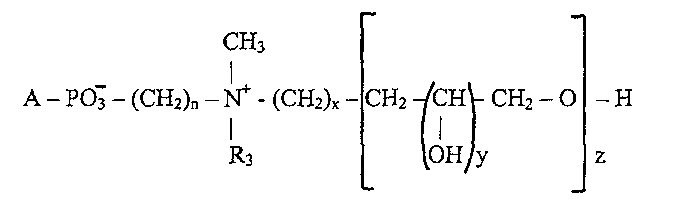 in which A is 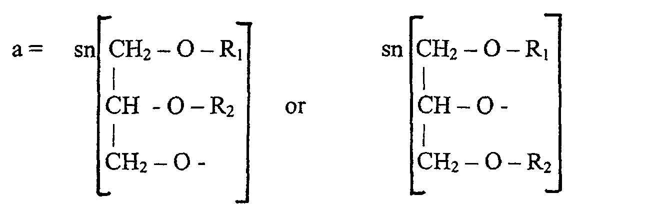 or 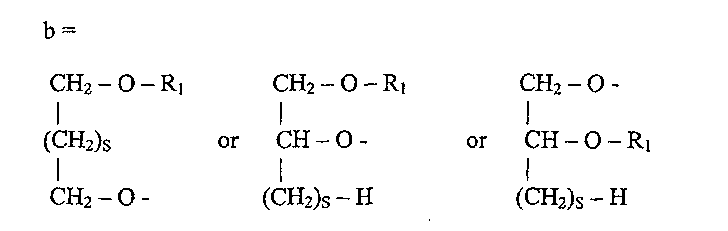 where R₁ and R₂ denote, independently of one another, hydrogen, a saturated or unsaturated acyl or alkyl residue which can be optionally branched or/and substituted wherein the sum of the carbon atoms in acyl and alkyl is 16 to 44 C atoms,
s is an integer from 0 to 8,
c = a residue of a primary or secondary alcohol of formula RO- where R denotes a saturated or unsaturated alkyl residue of 12 to 30 carbon atoms mainly with a cis double bond,
n represents an integer from 2 to 8,
R₃ a = can be 1,2 dihydroxypropyl or
b = can be alkyl with 1 to 3 C atoms, if z > 0 or
c = can be alkyl with 1 to 3 C atoms, when n ≠ 2 and z = 0,
x = represents an integer from 0 to 8,
y = 1 for z = 1 to 5 or
= is 1 to 4 for z = 1
z = is an integer from 0 to 5.

2. Compound as claimed in claim 1 with
x=0
y=1
z=1.

3. Compound as claimed in claim 1 with
x=0
y=1
z=2.

4. Compound as claimed in claim 1 with
x=0
y = 1
z=3.

5. Compound as claimed in claim 1 with
x=0
y=1
z=4.

6. Compound as claimed in claim 1 with
x = 1 - 3
z = 0.

7. Compound as claimed in claim 6 with
x = 1
z=0.

8. Compound as claimed in one of the previous claims with
n=2-6.

9. Compound as claimed in claim 8 with
n=2-4.

10. Compound as claimed in one of the previous claims, wherein R₃ is a CH₃ residue.

11. Compound as claimed in one of the previous claims,
**characterized in that**,
A is a group of formula a.

12. Compound as claimed in one of the claims 1 to 11,
**characterized in that**
A is a group of formula a and one of R₁ and R₂ is H or an alkyl chain with 1 - 3 C atoms.

13. Compounds as claimed in one of the previous claims,
**characterized in that**
A is a group of formula a and
n=3-6.

14. Compound as claimed in claim 13,
**characterized in that**,
n=3.

15. Compound as claimed in claim 13,
**characterized in that**
n=4.

16. Compound as claimed in one of the claims 1 to 10,
**characterized in that**
A is a group of formula B.

17. Compound as claimed in claim 11,
**characterized in that**
n=2.

18. Compound as claimed in one of the claims 1 to 10,
**characterized in that**
n=3.

19. Compound as claimed in one of the claims 1 to 10,
**characterized in that**
n=4.

20. Compound as claimed in one of the claims 1 to 10,
**characterized in that**
A is a group of formula c.

21. Compound as claimed in claim 20,
**characterized in that**
n=3.

22. Liposomes which contain phospholipids or/and alkylphospholipids, optionally cholesterol and 1 to 50 mole % of a compound of the general formula (I) of claim 1 or salts thereof, wherein the cholesterol, the phospholipids, the alkylphospholipids and the compound of formula (I) together amount to 100 mole %.

23. Use of a compound of the general formula (I) of claim 1 as a gene transport vehicle.

24. Use of a compound of the general formula (I) of claim 1 as a solvent for water-insoluble active substances.

25. Use as claimed in claim 24,
**characterized in that**
R₁ is a erucic acid residue.

26. Pharmaceutical composition,
**characterized in that**
it contains liposomes as claimed in claim 22 in which one or more pharmaceutically active substances are enclosed, optionally together with common pharmaceutical diluents, auxiliary substances, carriers and fillers.

## Revendications

1. Composé de formule générale (I) 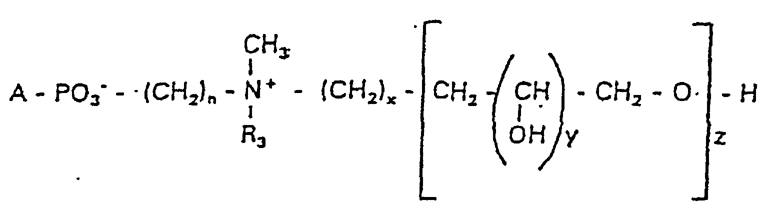 où A est 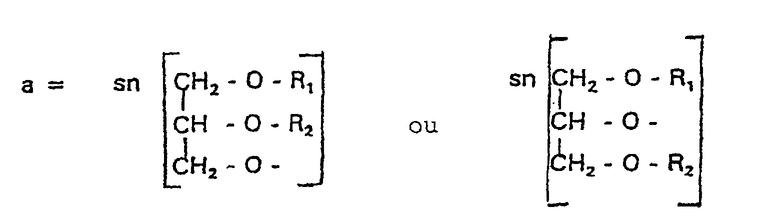 ou 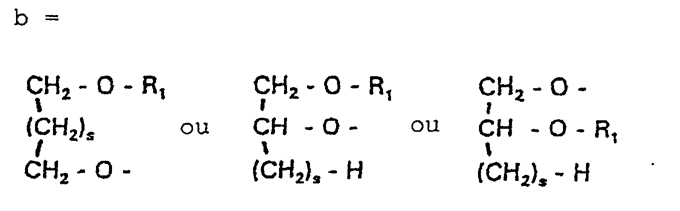 où R₁ et R₂ représentent indépendamment l'un de l'autre l'hydrogène, un reste acyle ou alkyle saturé ou insaturé, qui peut éventuellement être ramifié et/ou substitué, et la somme des atomes de carbone dans acyle et alkyle est de 16 à 44 atomes de carbone,
s représente un nombre entier de 0 à 8,
c = un reste d'un alcool primaire ou secondaire de formule RO-, où R représente un reste alkyle saturé ou insaturé principalement à double liaison cis, de 12 à 30 atomes de carbone,
n représente un nombre entier de 2 à 8,
R₃ peut être
a = 1,2-dihydroxypropyle ou peut être
b = alkyle de 1 à 3 atomes de carbone, quand z > 0 ou peut être
c = alkyle de 1 à 3 atomes de carbone, quand n ≠ 2 et z = 0,
x = représente un nombre entier de 0 à 8,
y = est 1 pour z = 1 à 5 ou
= est 1 à 4 pour z = 1
z = représente un nombre entier de 0 à 5.

2. Composé selon la revendication 1 avec
x = 0
y = 1
z = 1.

3. Composé selon la revendication 1 avec
x = 0
y = 1
z = 2.

4. Composé selon la revendication 1 avec
x = 0
y = 1
z = 3.

5. Composé selon la revendication 1 avec
x = 0
y = 1
z = 4.

6. Composé selon la revendication 1 avec
x = 1 - 3
z = 0.

7. Composé selon la revendication 6 avec
x = 1
z = 0.

8. Composé selon l'une des revendications précédentes avec
n = 2 - 6.

9. Composé selon la revendication 8 avec
n = 2 - 4.

10. Composé selon l'une des revendications précédentes où R₃ représente un reste CH₃.

11. Composé selon l'une des revendications précédentes **caractérisé en ce que** A représente un groupe de formule a.

12. Composé selon l'une des revendications 1 à 11 **caractérisé en ce que** A représente un groupe de formule a et, parmi R₁ et R₂, l'un est H ou une chaîne alkyle de 1 - 3 atomes de carbone.

13. Composé selon l'une des revendications précédentes **caractérisé en ce que** A représente un groupe de formule a et n = 3 - 6.

14. Composé selon la revendication 13
**caractérisé en ce que**
n = 3.

15. Composé selon la revendication 13
**caractérisé en ce que**
n = 4.

16. Composé selon l'une des revendications 1 à 10 **caractérisé en ce que** A représente un groupe de formule b.

17. Composé selon la revendication 11
**caractérisé en ce que**
n = 2.

18. Composé selon l'une des revendications 1 à 10 **caractérisé en ce que**
n = 3.

19. Composé selon l'une des revendications 1 à 10 **caractérisé en ce que**
n = 4.

20. Composé selon l'une des revendications 1 à 10 **caractérisé en ce que** A représente un groupe de formule c.

21. Composé selon la revendication 20
**caractérisé en ce que**
n = 3.

22. Liposomes qui contiennent des phospholipides et/ou des alkylphospholipides, éventuellement du cholestérol et 1 à 50 mol% d'un composé de formule générale (I) de la revendication 1 ou de ses sels, où le cholestérol, les phospholipides, les alkylphospholipides et le composé de formule (I) représentent ensemble 100 mol%.

23. Utilisation d'un composé de formule générale (I) de la revendication 1 comme véhicule de transport de gènes.

24. Utilisation d'un composé de formule générale (I) de la revendication 1 comme solvant pour principes actifs insolubles dans l'eau.

25. Utilisation selon la revendication 24 **caractérisée en ce que** R¹ représente un reste d'acide érucique.

26. Composition pharmaceutique **caractérisée en ce qu'**elle contient des liposomes selon la revendication 22 dans lesquels sont inclus un ou plusieurs principes actifs pharmaceutiques, éventuellement avec des diluants, adjuvants, supports et charges pharmaceutiquement courants.
